# EUROPEAN PATENT SPECIFICATION

(11) **EP 2 178 521 B1**
(45) Date of publication and mention of the grant of the patent: **15.01.2014**
(21) Application number: 08758272.2
(22) Date of filing: 11.07.2008
(51) Int. Cl.: A61K 9/68, A61K 9/20, A61K 31/495, A61K 31/496, A61K 47/40, A61K 47/48

(54) **STABLE MEDICATED CHEWING GUM COMPRISING CYCLODEXTRIN INCLUSION COMPLEX**
STABILES ARZNEIMITTELHALTIGES KAUGUMMI MIT EINEM CYCLODEXTRIN-EINSCHLUSSKOMPLEX
GOMME À MÂCHER MÉDICAMENTEUSE STABLE COMPRENANT UN COMPLEXE D'INCLUSION DE CYCLODEXTRINE

(30) Priority: 11.07.2007 WO PCT/DK2007/050094
(43) Date of publication of application: 28.04.2010
(73) Proprietor: Fertin Pharma A/S, 7120 Vejle (DK)
(72) Inventor: ANDERSEN, Carsten, DK-7100 Vejle (DK); LAO, My, Ly, DK-7120 Vejle Ø (DK); SZEMÁN, Julianna, H-1222 Budapest (HU); SZENTE, Lajos, H-1118 Budapest (HU)
(74) Representative: Østergaard, Steen
(86) International application number: PCT/DK2008/000264
(87) International publication number: WO 2009/006898

(56) References cited:
- WO-A-97/41843
- WO-A-2006/002622
- WO-A1-2009/007768
- WO-A1-2009/007769
- US-A1- 2005 038 039
- LOFTSSON T ET AL: "SOLUBILIZATION AND STABILIZATION OF DRUGS THROUGH CYCLODEXTRIN COMPLEXATION" ACTA PHARMACEUTICA NORDICA, ELSEVIER SCIENCE PUBLISHERS, AMSTERDAM, NL, vol. 3, no. 4, January 1991 (1991-01), pages 215-217, XP000612133 ISSN: 1100-1801

## Description

### FIELD OF THE INVENTION

The present invention is directed to medicament-containing chewing gums. In particular, the present invention is directed to chewing gums, which effectively stabilise compounds according to formula I contained therein and to methods for preparing such chewing gums.

### TECHNICAL BACKGROUND OF THE INVENTION

People suffering from hay fever, seasonal allergy, and allergy to other substances (such as dust mites, animal dander, and molds), including runny nose, sneezing, and red, itchy, tearing eyes, commonly take medications called antihistamines for symptomatic relief.

It is commonly accepted that one group of useful antihistamines are active compounds such as 2-[4-(diphenylmethyl)-1-piperazine] derivatives having the general formula I: 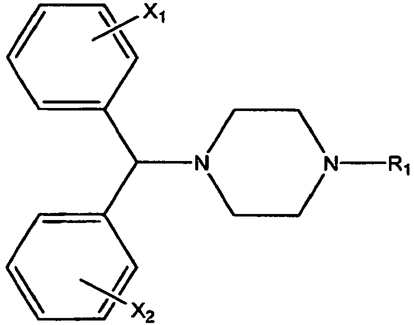 wherein
R₁ is selected from the group consisting of -CH₂CH₂-O-CH₂-R₂, -CH₂CH=CH-Ar₁, - CH₂-Ar₂ and 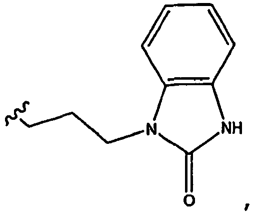
R₂ may be selected from the group consisting of a -CH₂OH group, a -COOH group and a -CONH₂ group;
Ar₁ and Ar₂ are independently an aromatic or heteroaromatic ring with 5 or 6 atoms in the ring, said heteroaromatic ring having 1, 2 or 3 heteroatoms selected from the group consisting of nitrogen, oxygen and sulfur, said ring being unsubstituted or substituted with C₁₋₄ alkyl, preferably methyl or tertiary butyl, Ar₁ and Ar₂ preferably being unsubstituted phenyl or phenyl substituted with C₁₋₄ alkyl, preferably methyl or tertiary butyl; and
X₁ and X₂ may independently be selected from the group consisting of a hydrogen atom, a halogen atom, a straight-chain or branched C₁-C₄ alkoxy group or a trifluoromethyl group; as well as pharmaceutically acceptable salts, geometrical isomers, enantiomers, diastereomers and mixtures thereof.

In the present context, the term "C₁₋₄-alkoxy" is intended to mean C₁₋₄-alkyl-oxy, such as methoxy, ethoxy, n-propoxy, isopropoxy, n-butoxy, isobutoxy, sec-butoxy and tert-butoxy. Furthermore, the term "halogen" includes fluorine, chlorine, bromine and iodine.

One of the most popular and effective antihistamines is cetirizine, which in its dihydrochloride form marketed under the tradename Zyrtec^{®}, the (S)-enantiomer thereof, levocetirizine, in its dihydrochloride form marketed under the trade name Xyzal^{®} and efletirizine in its dihydrochloride form.

A serious problem encountered with the administration of these active compounds in an oral composition is the loss of stability which is observed during production and storage of the chewing gum. Therefore, attempts have been made to identify the reason for this loss of stability and subsequent to find methods for counteracting this problem.

In US 2005/0038039 it is speculated that the loss of stability is caused by the interaction of active compound of formula I with specific polyols used in oral formulations for masking the bitter taste of the active compound. Thus, US 2005/0038039 discloses an oral pharmaceutical composition containing two separate formulations: a first formulation, which contains an active compound according to the above formula I and which first formulation does not contain polyols having a molecular weight of less than 300 g/mol in a molar ratio between the polyol and active compound of formula I above 10; and a second formulation, which contains one or more polyol(s) with a molecular weight of less than 3000 and which second formulation is free of any drug.

### SUMMARY OF THE INVENTION

Thus, one object of the present invention is to provide chewing gums containing a compound according to formula I wherein the stability of the compound(s) according to formula I is improved. It is furthermore one object of the invention to provide methods for producing the stable chewing gum containing the compound(s) according to formula I.

During their study of the cause of the stability problem mentioned in US 2005/0038039, the present inventors surprisingly discovered that these problems could be solved by disregarding the teaching of US 2005/0038039 and instead using the active compound in the form of a cyclodextrin inclusion complex.

Accordingly, the present invention provides a chewing gum comprising a gum base and at least one inclusion complex comprising a cyclodextrin and an active compound selected from 2-[4-(diphenylmethyl)-1-piperazine] derivatives having the general formula I: 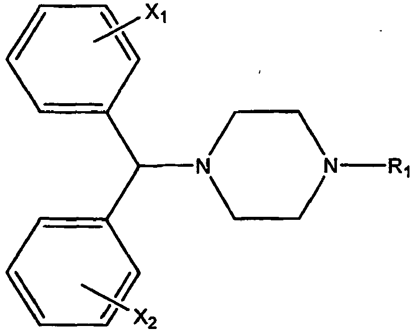 wherein
R₁ is selected from the group consisting of -CH₂CH₂-O-CH₂-R₂, -CH₂CH=CH-Ar₁, -CH₂-Ar₂ and 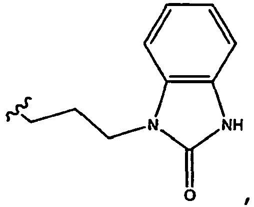
R₂ may be selected from the group consisting of a -CH₂OH group, a -COOH group and a -CONH₂ group;
Ar₁ and Ar₂ are independently an aromatic or heteroaromatic ring with 5 or 6 atoms in the ring, said heteroaromatic ring having 1, 2 or 3 heteroatoms selected from the group consisting of nitrogen, oxygen and sulfur, said ring being unsubstituted or substituted with C₁₋₄ alkyl, preferably methyl or tertiary butyl, Ar₁ and Ar₂ preferably being unsubstituted phenyl or phenyl substituted with C₁₋₄ alkyl, preferably methyl or tertiary butyl, and
X₁ and X₂ may independently be selected from the group consisting of a hydrogen atom, a halogen atom, a straight-chain or branched C₁-C₄ alkoxy group or a trifluoromethyl group; as well as pharmaceutically acceptable salts, geometrical isomers, enantiomers, diastereomers and mixtures thereof.

The present inventors have additionally found that active compounds according to formula I may tend to stick to the gum base of the chewing gum, thereby undesirably slowing down, or even prohibiting, the complete release of the active compound from the chewing gum. This problem is now solved by the present invention which provides a faster and more complete release of the active compound.

In a further aspect, the present invention relates to a method of preparing a chewing gum comprising at least one inclusion complex comprising a cyclodextrin and one or more active compound(s) according to formula I, the method comprising the steps of: providing at least one inclusion complex comprising a cyclodextrin and a compound according to formula I; a) providing a gum base; b) optionally providing one or more further chewing gum ingredients; c)mixing said inclusion complex and gum base, and optionally the one and more further chewing gum ingredients, and thus obtaining a mixture; and d) shaping the mixture to obtain the chewing gum.

In a still further aspect, there is provided a method of preparing a compressed chewing gum comprising at least one inclusion complex comprising a cyclodextrin and one or more active compound(s) according to formula I having one compressed module, the method comprising the steps of: a) providing a portion comprising at least one inclusion complex comprising a cyclodextrin and one or more active compound(s) according to formula I, and chewing gum particles containing gum base; b) optionally providing one or more further chewing gum ingredients; c) dosing the portion comprising the at least one inclusion complex comprising a cyclodextrin and one or more active compound(s) according to formula I, and chewing gum particles containing gum base, and optionally the one or more further chewing gum ingredients; and d) compressing a) and b) after dosing to obtain a first compressed module.

A further aspect of the present invention relates to a method of preparing a compressed chewing gum comprising at least one inclusion complex comprising a cyclodextrin and one or more active compound(s) according to formula I having one compressed module, the method comprising the steps of: a) providing a portion comprising at least one inclusion complex comprising a cyclodextrin and a compound according to formula I, and chewing gum particles containing gum base; b) optionally providing one or more further chewing gum ingredients; c) mixing the portion comprising at least one inclusion complex comprising a cyclodextrin and a compound according to formula I, and the chewing gum particles containing gum base, and optionally the one or more further chewing gum ingredients, thus obtaining a mixture; and d) compressing the mixture, to obtain a first compressed module.

In an even further aspect, the present invention relates to a method of preparing a chewing gum comprising at least one inclusion complex comprising a cyclodextrin and one or more active compound(s) according to formula I having two compressed modules, the method comprising the steps of: a) providing chewing gum particles containing gum base and optionally portion(s) comprising one or more chewing gum ingredients; b) providing a portion comprising tablet material and a portion comprising at least one inclusion complex comprising a cyclodextrin and one or more active compound(s) according to formula I; c) compressing a), to obtain a first compressed module; d) contacting the first compressed module with b); e) compressing b) and the first compressed module, to obtain a coherent compressed chewing gum comprising a first compressed module and a second compressed module.

Furthermore, an aspect of the present invention relates to a method of preparing a compressed chewing gum comprising at least one inclusion complex comprising a cyclodextrin and one or more active compound(s) according to formula I having three compressed modules, the method comprising the steps of: a) providing chewing gum particles containing gum base, a portion comprising at least one inclusion complex comprising a cyclodextrin and a compound according to formula I, and optionally portion(s) comprising one or more chewing gum ingredients; b) providing a portion comprising tablet material and optionally a portion comprising at least one inclusion complex comprising a cyclodextrin and a compound according to formula I; c) providing a portion comprising tablet material and a portion comprising at least one inclusion complex comprising a cyclodextrin and a compound according to formula I; d) locating b) and c) on opposite sites of a) following a sequence of one or more compressing step(s), to obtain a coherent compressed chewing gum tablet comprising a first compressed module and a second compressed module and a third compressed module.

In one aspect, there is provided a method for improving the stability of one or more active compound(s) according to formula I contained in a medicated chewing gum, comprising the steps of complex binding said compound to a cyclodextrin.

A further aspect of the present invention relates to the use of cyclodextrin to protect one or more active compound(s) according to formula I against degradation in a chewing gum, wherein said cyclodextrin forms an inclusion complex with said compound(s).

Furthermore, an aspect relates to the use of an inclusion complex comprising a cyclodextrin and one or more active compound(s) according to formula I in a mediated chewing gum comprising the compound(s) according to formula I for improving the stability of said compound(s).

A final aspect of the present invention relates to the use of a cyclodextrin in a chewing gum comprising one or more active compound(s) according to formula I, wherein at least 80% of the initial amount of said compound(s) is left after storage for 1 month at a temperature of 40°C and a humidity of 75% RH, wherein said cyclodextrin forms an inclusion complex with said compound(s).

### BRIEF DESCRIPTION OF THE FIGURES

The invention will now be described with reference to the drawings of which
Figs. 1a-1b illustrate a two-layer compressed tablet according to an embodiment of the invention,
Figs. 2a-2b illustrate a three layer compressed tablet according to an embodiment of the invention,
Figs. 3a-3b illustrate a further two layer compressed tablet according to an embodiment of the invention,
Figs. 4a-4b illustrate a further two layer compressed tablet according to an embodiment of the invention, and where
Figs. 5a-5b illustrate a further two layer compressed tablet according to an embodiment of the invention.

### DETAILED DESCRIPTION OF THE INVENTION

### Definitions

In the context of the present invention, the term "inclusion complex" relates to molecular aggregate resulting from binding a molecule, e.g. a compound according to formula I, to a cyclodextrin. Without being bound by theory it is believed that at least a part of the molecule is located in the hydrophobic cavity of the cyclodextrin, and the molecule is possibly bound to the inner, hydrophobic cavity by Van der Waals forces.

The term "complex-bound compound according to formula I" relates in the present context to any compound according to formula I which forms part of an inclusion complex with one or more cyclodextrin(s).

The term "complex-bound cyclodextrin" means in the present context any cyclodextrin which forms part of an inclusion complex with one or more active compounds according to formula I.

In the present context, the term "gum base" refers in general to a commercially available gum base suitable for production of chewing gum. Such gum bases normally comprise one or more elastomeric compounds which may be of synthetic or natural origin, one or more resinous compounds which may be of synthetic or natural origin and softening compounds.

The term "gum base composition" as used herein may be a gum base as defined above comprising one or more ingredients (e.g. sweetener, flavour, colouring agents, fillers etc.) as described below.

The term "chewing gum composition" is the final formulation, which constitutes at least a part of the chewing gum ready for sale or use by the consumer. A chewing gum composition may comprise an inclusion complex comprising a cyclodextrin and a compound according to formula I, sweetener and/or flavour and optionally other ingredients like colouring agents, enzymes, humectants, flavour enhancers, anticaking agents etc.

Furthermore, the expression "compressed chewing gum tablet" denote a ready for use chewing gum, e.g. comprising compressed particles containing gum base possibly mixed with an inclusion complex comprising a cyclodextrin and a compound according to formula I, sweeteners, flavour or other ingredients and optionally coated. As described in detail below, a compressed chewing gum tablet is produced by an initial conventional mixing of the gum base with e.g. water-insoluble ingredients such as elastomers and resins, followed by a granulation or the like of the obtained gum base mix. The obtained particles containing gum base may then be mixed with further chewing gum ingredients, such as an inclusion complex comprising a cyclodextrin and a compound according to formula I, sweeteners and flavours. The final mix may then be compressed under high pressure (typically when applying cooling) into to a compressed chewing gum tablet or a compressed module.

Thus, the expression "chewing gum particles containing gum base" refers to particulated material of chewing gum composition and is to be understood as any form of chewing gum particles containing a certain amount of gum base as described in detail below. The chewing gum particles may be in any suitable form such as pellets, granules, agglomerates, powder. Thus, in some embodiments, the particles have been particulated prior to application. Particulation may be in any form of "building up" particles from smaller primary particles into macro particles or in any form of "building down" from larger substances into macro particles. Any form of particulation may be applied, such as granulation, pelletizing, agglomeration, or any other suitable means for particulation, as described below. Thus, the particles may also to be understood as macroparticles. The components of the chewing gum composition are described in detail below. More details regarding the composition of compressed chewing gum and methods of preparing it may be found in WO 2004/068 964, WO 2004/004480, and WO 2004/004 479.

Furthermore, the expression "compressed chewing gum particles containing gum base" refers to a portion of chewing gum particles which become compressed after mixed with e.g. an inclusion complex comprising a cyclodextrin and a compound according to formula I, sweeteners or flavours.

### Preferred embodiments

The present invention relates to a medicated chewing gum with an effective amount of a compound according to formula I, having a chemical stability of the active medicament during production, storage and chewing which allows the chewing gum to be a suitable carrier for said medicament. As mentioned above, it was first after having identified that an oxidation caused by chewing gum ingredients is the reason of the diminishing stability of the active medicament observed during production, storage and chewing, it was possible to provide a method for counteracting this instability.

Thus, in efforts to improve the chemical stability of the active compound according to formula I in chewing gums, research has led to a new chemical entity, an inclusion complex between cyclodextrin and one or more active compound(s) according to formula I. Accordingly, the invention provides a chewing gum comprising a gum base and at least one inclusion complex comprising a cyclodextrin and one or more active compound(s) selected from 2-[4-(diphenylmethyl)-1-piperazine] derivatives having the general formula I: 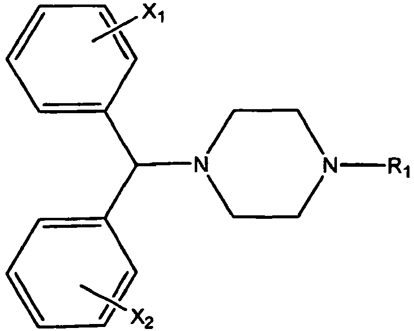 wherein
R₁ is selected from the group consisting of -CH₂CH₂-O-CH₂-R₂, -CH₂CH=CH-Ar₁, -CH₂-Ar₂ and 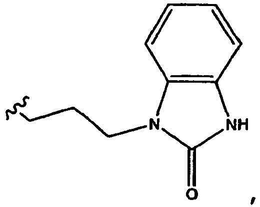
R₂ may be selected from the group consisting of a -CH₂OH group, a -COOH group and a -CONH₂ group;
Ar₁ and Ar₂ are independently an aromatic or heteroaromatic ring with 5 or 6 atoms in the ring, said heteroaromatic ring having 1, 2 or 3 heteroatoms selected from the group consisting of nitrogen, oxygen and sulfur, said ring being unsubstituted or substituted with C₁₋₄ alkyl, preferably methyl or tertiary butyl, Ar₁ and Ar₂ preferably being unsubstituted phenyl or phenyl substituted with C₁₋₄ alkyl, preferably methyl or tertiary butyl, and
X₁ and X₂ may independently be selected from the group consisting of a hydrogen atom, a halogen atom, a straight-chain or branched C₁-C₄ alkoxy group or a trifluoromethyl group; as well as pharmaceutically acceptable salts, geometrical isomers, enantiomers, diastereomers and mixtures thereof.

In accordance with the present invention the active compound according to the formula I is complex-bound to a cyclodextrin which results in an improved chemical stability of the active compound during production, storage and chewing compared to a compound according to formula I not complex-bound to a cyclodextrin and stored under the same conditions. Thus, in one embodiment of the present invention, the chewing gum is one wherein the stability of the compound(s) according to formula I is improved by at least 4% by weight when stored for 1 month at a temperature of 40°C and a humidity of 75% RH compared to a chewing gum comprising a compound according to formula I not complex-bound to a cyclodextrin and stored under the same conditions. In further embodiments, the chewing gum is one wherein the stability of the compound(s) is improved by at least 5% by weight, such as at least 6%, including at least 10%, such as at least 15% by weight when stored for 1 month at a temperature of 40°C and a humidity of 75% RH compared to a chewing gum comprising a compound according to formula I not complex-bound to a cyclodextrin and stored under the same conditions.

However, in a preferred embodiment of the present invention, the chewing gum is one wherein at least 80% of the initial amount of the compound(s) according to formula I is left after storage for 1 month at a temperature of 40°C and a humidity of 75% RH. In a particular interesting embodiment of the invention at least 85%, preferably at least 90%, most preferably at least 95%, most preferably 99% of the initial amount of the compound(s) according to formula I is left after storage for 1 month, for 2 months or even for 3 months at a temperature of 40°C and a humidity of 75% RH.

### Compound according to formula I

The active compounds according to formula I are preferably orally active and selective histamine H₁-receptor antagonists. In preferred embodiments of the invention, the one and more active compound(s) according to formula I are so selected that X₁ is a hydrogen atom, X₂ is a halogen atom, and R₁ is -CH₂CH₂-O-CH₂-COOH. Preferably, the active compound according to formula I is a salt, e.g. a dihydrochloride salt.

In another embodiment of the invention, the active compound according to formula I is selected from the group consisting of buclizine, cetirizine, chlorcyclizine, cinnarizine, cyclizine, hydroxyzine, levocetirizine, meclozine, efletirizine and oxatomide, as well as any pharmaceutically acceptable salts, geometrical isomers, enantiomers, diastereomers and mixtures thereof.

Useful active compound(s) according to formula I may be selected from the group consisting of cetirizine, the dihydrochloride salt of cetirizine, levocetirizine, the dihydrochloride salt of levocetirizine, efletirizine, and the dihydrochloride salt of efletirizine.

In a preferred embodiment, the active compound according to formula I is cetirizine, i.e. 2-[2-[4-[(4-chlorophenyl)-phenyl-methyl] piperazin-1-yl]ethoxy]acetic acid having the following formula II: 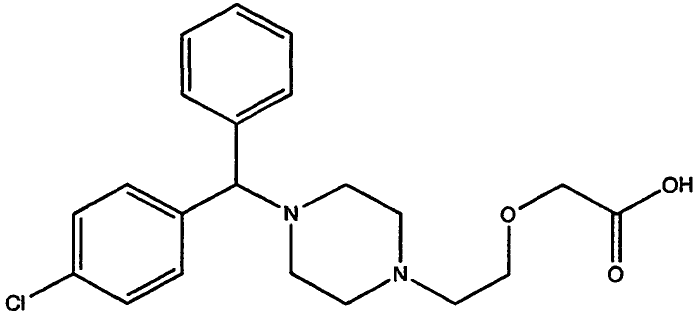 or a salt thereof, preferably citerizine dihydrochloride.

In embodiments of the invention, the chewing gum comprises the compound(s) according to formula I, e.g. cetirizine or cetirizine dihydrochloride, in an amount in the range of 0.1 - 50 mg, preferably in the range of 1 - 30 mg, and even more preferably in the range of 5-25 mg.

### Type of cyclodextrin,

The cyclodextrin may be selected from alpha-cyclodextrin (α-cyclodextrin), beta-cyclodextrin (β-cyclodextrin), gamma-cyclodextrin (γ-cyclodextrin), i.e. the 6-, 7-, or 8- sugar unit macrocycle, respectively, and derivatives thereof. A preferred embodiment of the present invention is wherein the cyclodextrin is β-cyclodextrin. Without being bound by theory, it is believed that due to the appropriate ring size of β-cyclodextrin creating a cone shape surrounding the active compound according to formula I a suitable protection against oxidation or other stability-reducing actions is obtained.

The cyclodextrin useful in the present invention may be modified such that some or all of the primary or secondary hydroxyls of the macrocyle, or both, may be alkylated or acylated. Methods of modifying these alcohols are well known to the person skilled in the art and many derivatives are commercially available. The cyclodextrin may be modified such that one or more of the primary or secondary hydroxyls of the macrocyle, or both, may be alkylated or acylated. Methods of modifying these alcohols are well known to the person skilled in the art and many are commercially available. Thus, some or all of the hydroxyls of cyclodextrin may have be substituted with an O-R group or an O-C(O)-R, wherein R is an optionally substituted C₁₋₆ alkyl, an optionally substituted C₂₋₆ alkenyl, an optionally substituted C₂₋₆ alkynyl, an optionally substituted aryl or heteroaryl group. R may be methyl, ethyl, propyl, butyl, pentyl, or hexyl group. Consequently, O-C(O)-R may be an acetate. Furthermore, R may be such as to derivatize cyclodextrin with the commonly employed 2-hydroxyethyl group, or 2-hydroxypropyl group. Moreover, the cyclodextrin alcohols may be per-benzylated, per-benzoylated, or benzylated or benzoylated on just one face of the macrocycle, or wherein only 1, 2, 3, 4, 5, or 6 hydroxyls are benzylated or benzoylated. The hydroxyl groups of cyclodextrin may be per-alkylated or per-acylated such as per-methylated or peracetylated, or alkylated or acylated, such as methylated or acetylated, on just one face of the macrocycle, or wherein only 1, 2, 3, 4, 5, or 6 hydroxyls are alkylated or acylated, such as methylated or acetylated. In useful embodiments, at least one cyclodextrin is selected from the group consisting of heptakis (2,6-di-o-methyl)-beta-cyclodextrin, randomly methylated beta-cyclodextrin and hydroxypropyl beta-cyclodextrin.

In a preferred embodiment, the chewing gum is one wherein at least one the cyclodextrin is beta-cyclodextrin.

### Location of the inclusion complex

One advantage of the present invention is that the inclusion complex can be used in any type of chewing gum and be located at different places in the chewing gum. This leaves the chewing gum industry with a high flexibility in the production of different kinds of medicated chewing gum, without compromising the stability of the active compound. Thus, useful chewing gum types include chewing gum comprising a center-filled or chewing gum processed into a number of different shapes such as a stick, a core, a tablet, a slab, a bead, a pellet, a tape, or a ball. Furthermore, the chewing gum according to the invention may also be a compressed chewing gum or a compressed chewing gum tablet comprising an inclusion complex and one or more compressed module(s) comprising compressed chewing gum particles containing gum base. Such a chewing gum tablet may in accordance with the invention be processed into in a number of different shapes such as a stick, a core, a tablet, a slab, a bead, a pellet, a tape, or a ball.

A useful embodiment of the present invention is one wherein the chewing gum comprises a first layer compressed module comprising compressed chewing gum particles containing gum base. One advantage of compressing the chewing gum particles containing gum base together with an inclusion complex comprising cyclodextrin and one or more active compound(s) according to formula I as described herein is that the active compound is released faster and more complete than from conventionally mixed chewing gum, or from compressed chewing gum in which the one or more active compound(s) according to formula I are not present in the form of an inclusion complex.

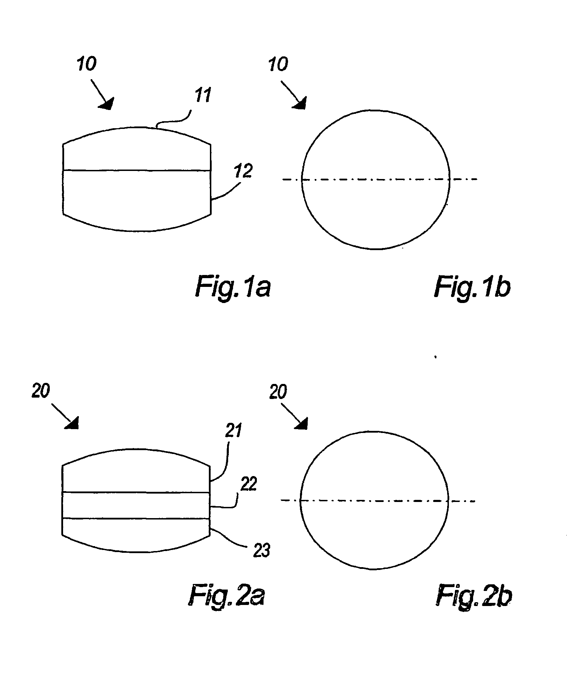

In an embodiment of the invention, the chewing gum is one wherein the first compressed module is on top of a second compressed module. Figure 1a and 1b illustrates such a chewing gum according to the invention. The illustrated chewing gum 10 comprises two chewing gum modules 11 and 12.

According to the illustrated embodiment, each module is simply comprised by a layer. The multi-module chewing gum may in this embodiment be regarded as a two-layer or a two-module chewing gum 10. The two modules 11 and 12 are adhered to each other. Different processes may be applied for the purpose as described below. However, according to a preferred embodiment of the invention, the mutual adhering between the two modules is obtained by the compression of one module 11 onto the other module 12.

The illustrated chewing gum 10 may for example comprise a non-gum base-containing module 11 and a gum base-containing module 12. Thus, in an embodiment of the invention, the chewing gum is one wherein the second compressed module, i.e. the module 11, comprises compressed tablet material. Examples of useful tablet material are described below.

However, it may under some circumstances be useful also to include gum base in the second compressed module. Thus, in a useful embodiment, the second compressed module, i.e. the module 11, comprises compressed chewing gum particles containing gum base and optionally one or more further chewing gum ingredients.

Figure 2a illustrates a cross-section of a compressed chewing gum according to the invention and figure 2b illustrates the chewing gum from above. Thus, an embodiment of the present invention is one wherein the first compressed module is on top of a second compressed module on top of a third compressed module, or as described in another manner, the first module is located between two outer modules.

The illustrated chewing gum tablet 20 in figure 2a may in one embodiment comprises a three-module chewing gum of which the lowest module or third module 23 comprises compressed tablet material, and the module 21 and 22 are as described above in figure 1. In a further embodiment, the compressed chewing gum tablet 20 is one wherein the third compressed module 23 comprises compressed chewing gum particles containing gum base and optionally one or more further chewing gum ingredients. The module 21 and 22 may be as described above in figure 1.

However, under some circumstances it may be useful to provide a chewing gum having three modules comprising compressed chewing gum particles containing gum base. Thus, in a useful embodiment, the compressed chewing gum tablet is one wherein said first, second and/or third compressed module comprises compressed chewing gum particles containing gum base and one or more further chewing gum ingredients.

An interesting embodiment of the invention is where the chewing gum 20 comprises three modules, wherein the first compressed module 22 comprises compressed chewing gum particles containing gum base and one or more further chewing gum ingredients, and where said module is located between two compressed outer modules 21 and 23 comprising compressed tablet material.

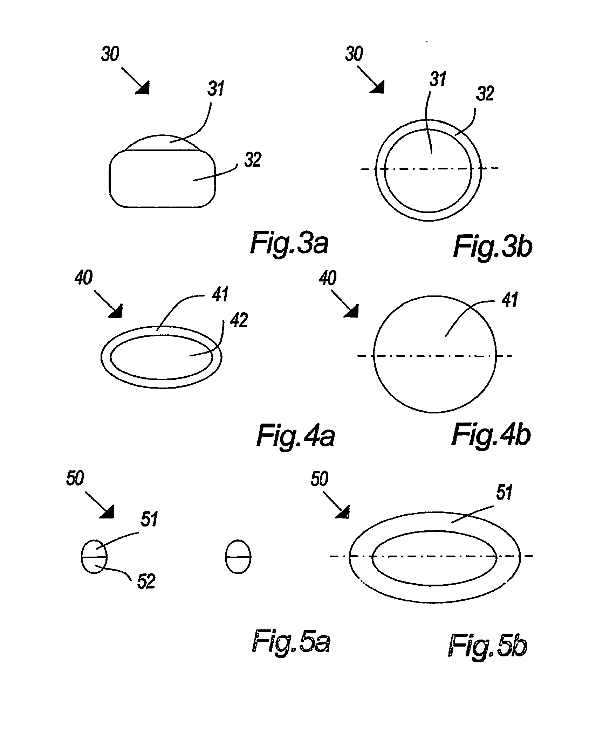

Figure 3a illustrates a cross-section of a compressed chewing gum 30 according the invention and illustrated in figure 3b from above. The illustrated chewing gum 30 comprises a module 32 comprising compressed chewing gum particles containing gum base and optionally one or more further chewing gum ingredients upon which a second module 31 is arranged. The module 31 may comprise compressed tablet material or compressed chewing gum particles containing gum base and one or more further chewing gum ingredients.

Figure 4a illustrates a cross-section of a further compressed multi-modular chewing gum 40 according to the invention and illustrated in figure 4b from above. The chewing gum 40 differs somewhat from the other described chewing gums in the sense that the chewing gum comprises a module 42 comprising compressed chewing gum particles containing gum base and optionally one or more further chewing gum ingredients forming a gum center. The module 42 is encapsulated by a surrounding module 41. The module 41 may comprise compressed tablet material or compressed chewing gum particles containing gum base and one or more further chewing gum ingredients.

Figure 5a illustrates a cross-section of a compressed multi-modular chewing gum 50 according to the invention and illustrated in figure 5b from above. According to the illustrated embodiment, showing a ring-formed two layer tablet 50, a module 52 comprising compressed chewing gum particles containing gum base at a certain concentration and optionally one or more further chewing gum ingredients, whereas the other layer 51 comprises compressed tablet material.

Alternatively, the chewing gum module 51 may comprise a content of compressed chewing gum particles containing gum base differing from that of the content of module 52, thereby facilitating a chewing gum providing at least two different release profiles in one piece.

In a useful embodiment, the chewing gum of the present invention is one comprising a first and a second compressed module, wherein the first compressed module comprises compressed chewing gum particles containing gum base and an inclusion complex comprising cyclodextrin and one or more active compound(s) according to formula I.

It is well known for a skilled person in the art to prepare a chewing gum with multiple compressed modules. In accordance with invention, the second compressed module may preferable not comprise gum base, or only comprise at most 1% gum base, such as at the most 0.5% gum base by weight of the second compressed module. In a preferred embodiment, the second module may comprise tablet material. Examples of useful tablet materials are described below.

In a further embodiment, the chewing gum is one comprising a first and a second compressed module, wherein the first compressed module comprises compressed chewing gum particles containing gum base, and the second compressed module comprises an inclusion complex comprising cyclodextrin and one or more active compound(s) according to formula I. Optionally, the second compressed module may further comprise tablet material. Besides good stability properties with respect to the active compound according to formula I, this embodiment also appears to provide a fast and efficient release of the active compound.

In an embodiment, the chewing gum comprises a first, a second and a third compressed module, wherein the first compressed module comprises compressed chewing gum particles containing gum base, and the second compressed module comprises an inclusion complex comprising cyclodextrin and one or more active compound(s) according to formula I and the third compressed module comprises tablet material. In a further embodiment, the chewing gum in one, wherein the first compressed module comprises further an inclusion complex comprising cyclodextrin and one or more active compound(s) according to formula I.

In a further embodiment of the present invention, the chewing gum is one wherein the first compressed module comprises compressed chewing gum particles containing gum base, and where said module is located between two compressed outer modules comprising tablet material and an inclusion complex comprising cyclodextrin and one or more active compound(s) according to formula I.

In accordance with the invention, inclusion complex comprising cyclodextrin and one or more active compound(s) according to formula I are compressed together with the chewing gum particles containing gum base. However, this process is described in detail below.

An interesting embodiment is where the chewing gum comprises a mixture comprising the at least one inclusion complex comprising a cyclodextrin and a compound according to formula I, and the gum base.

In a useful embodiment, the at least one inclusion complex is present in the coating of the chewing gum according to the invention. It will be understood, that it under some circumstances may be useful to place the inclusion complex only in the coating, whereas it sometimes may be useful to place the inclusion complex both in the chewing gum and in the coating. Examples of useful coatings are described below.

### Ratio between active compound and cyclodextrin in the inclusion complex

One or more molecules of the active compounds according to formula I may be included into the cavity of the cyclodextrin molecule. Conversely, one molecule of the active compound may be included into the cavity of one or more cyclodextrin molecules. The inclusion complex may exist in a variety of stoichiometric ratios. The stoichiometric ratio between the active compound and the cyclodextrin is dependent on a variety of physical factors during the formation of the inclusion complex. Furthermore, the stoichiometric ratio of the inclusion complex may be transitional and vary during its preparation. Given the inclusion of the active compound can result from a variety of interactions with any number of functional groups or moieties of the active compound, the depth at which the active compound is included within the cavity of a cyclodextrin may vary. Furthermore, the size of the cavity, which depends on the selection of cyclodextrin (α-cyclodextrin, β-cyclodextrin or γ-cyclodextrin) and on whether the numerous free hydroxyl groups present on the periphery of the cavity of a cyclodextrin molecule are partially or fully derivatized, will influence the ability for the active compound to include itself into the cavity. These factors, amongst others, influence the molar ratio of the inclusion complex.

Given the above-stated factors, and that the moiety of the active compound molecule which may include itself into the cyclodextrin molecule may vary, the stoichiometric ratio, in a diluted solution, between the active compound according to formula I and the cyclodextrin of said inclusion complex, may be at the most 1:1, such as at the most 1:2 or 1:3. Preferably, at least one inclusion complex has a stoichiometric ratio between the compound(s) according to formula I and cyclodextrin selected from the group consisting of 1:1, 1:2, 2:3 or 1:3. For example, one molecule of active compound, or a moiety thereof, is at least partially inserted into the cavity of one cyclodextrin molecule or one molecule of active compound or moieties thereof, is at least partially inserted into the cavity of two molecules of cyclodextrin.

The formation of supramolecular non-covalent aggregates from the non-occupied cyclodextrins and from those occupied by one or more active compound(s) according to formula I will further increase the number of co-existing complex species with different stoichiometries in the formulations of matter. The aqueous systems containing active compound and any of the α-cyclodextrin, β-cyclodextrin or γ-cyclodextrin will have around 8-15 % out of the total empty cyclodextrin species in an aggregated form stabilized by intermolecular hydrogen bonds. These aggregates will have an average aggregate size of 20-50 nm as described by Coleman (1992).

### Combinations of different inclusion complexes with different stoichiometry

It will be understood that in solution and thus within a chewing gum, different inclusion complexes may exits having different stoichiometric ratio between the active compound according to formula I and the cyclodextrin. In one useful embodiment, the chewing gum is one wherein at least one inclusion complex has a stoichiometric ratio of 1:1 between the compound(s) according to formula I and cyclodextrin, and at least one inclusion complex has a stoichiometric ratio of 1:2 between the compound(s) according to formula I and cyclodextrin. In addition, the chewing gum may furthermore comprise at least one inclusion complex having a stoichiometric ratio of 1:3 between the compound(s) according to formula I and cyclodextrin.

In one interesting embodiment, the chewing gum is one wherein at least one inclusion complex has a stoichiometric ratio of 1:1 between the compound(s) according to formula I and cyclodextrin, and at least one inclusion complex has a stoichiometric ratio of 2:3 between the compound(s) according to formula I and cyclodextrin.

In preferred embodiments, the chewing gum is one wherein at most 75% by mole of the inclusion complexes have a stoichiometric ratio of 1:1 between the compound(s) according to formula I and cyclodextrin, and at least 25% by mole of the inclusion complexes have a stoichiometric ratio of 1:2, 1:3 or 2:3 between the compound(s) according to formula I and cyclodextrin.

In further embodiments, the chewing gum is one wherein at most 50% by mole of the inclusion complexes have a stoichiometric ratio of 1:1 between the compound(s) according to formula I and cyclodextrin, and at least 50% by mole of the inclusion complexes have a stoichiometric ratio of 1:2, 1:3 or 2:3 between the compound(s) according to formula I and cyclodextrin.

In still further embodiments, the chewing gum is one wherein at most 25% by mole of the inclusion complexes have a stoichiometric ratio of 1:1 between the compound(s) according to formula I and cyclodextrin, and at least 75% by mole of the inclusion complexes have a stoichiometric ratio of 1:2, 1:3 or 2:3 between the compound(s) according to formula I and cyclodextrin.

In further embodiments, the chewing gum is one wherein at most 10% by mole of the inclusion complexes have a stoichiometric ratio of 1:1 between the compound(s) according to formula I and cyclodextrin, and at least 90% by mole of the inclusion complexes have a stoichiometric ratio of 1:2, 1:3 or 2:3 between the compound(s) according to formula I and cyclodextrin.

Methods for analysing the different stoichiometric ratio within an inclusion complex are known by the skilled person. For example, if the inclusion complex is in a solid state, solid phase NMR may be used. However, if the inclusion complex is in solution electro-spray MS method (ES/MS-technique) may be used where it is possible to differentiate between the different stoichiometric ratios due to their different times of flight Mass values. Furthermore, diffusion kinetic studies in water solution could be performed that would indirectly indicate the co-existence of different 1:1, 1:2, 1:3, 2:3 molar ratios.

### Free cetirizine and cyclodextrin

In addition to the inclusion complex, the chewing gum according to the invention may comprise one or more free active compound(s) according to formula I, i.e. molecules of the active compound which do not form an inclusion complex with one or more cyclodextrin molecules.

In the contrary, it may be useful to shift the equilibrium process toward complexed status of the compound(s) according to formula I and thus to use more cyclodextrin molecules than actually needed for an equimolar amount. Accordingly, in a useful embodiment the chewing gum according to the invention further comprises free cyclodextrin, i.e. cyclodextrin molecules which do not form an inclusion complex with a compound according to formula I.

In an interesting embodiment, the chewing gum according to the invention is one which further comprises free cyclodextrin and free compound according to formula I.

### Amount of inclusion complex

The inclusion complex comprising cyclodextrin and one or more active compound(s) according to formula I may be present in an amount of at least 0.2% by weight of the uncoated chewing gum. In useful embodiments, the inclusion complex is present in an amount of at least 0.5%, 1%, 2.5%, 5%, 7.5%, 10%, 15%, 20%, or 25% by weight of the uncoated chewing gum.

In a preferred embodiment, the inclusion complex is present in the chewing gum according to the invention in an amount in the range 0.2% to 25%, such as in the range of 0.5% to 7.5%, including in the range of 0.7% to 6%, e.g. in the range of 1% to 4%, including in the range of 1% to 10%, such as in the range of 5% to 10%, including in a range of 7.5% to 15% by weight of the uncoated chewing gum.

The amount present of the inclusion complex in the chewing gum may also be calculated relative to the gum base. Thus, in useful embodiments, the inclusion complex is present in an amount of at least 1%, 2.5%, 5%, 10%, 15%, 20%, 25%, 30%, or 50% by weight of the gum base.

In preferred embodiments, the inclusion complex is present in the chewing gum according to the invention in an amount in the range of 1% to 50% by weight of the gum base, such as in the range of 2.5% to 30%, including in the range of 5% to 25%, e.g. in the range of 10% to 20%, such as in the range of 10% to 30%, including in the range of 15% to 20%, such as in the range of 20% to 30%, including in a range of 25% to 50% by weight of the gum base.

### Ratio between active compound and cyclodextrin

In a suitable embodiment of the present invention, the chewing gum is one wherein the molar ratio between the total amount of active compound according to formula I and the total amount of cyclodextrin in the chewing gum is at the most 1:1, respectively. Preferably, a molar ratio of at the most 1:2, 1:3, 1:4, 1:5, 1:7, 1:8, or 1:10 exits between the total amount of active compound according to formula I and the total amount of cyclodextrin.

Alternatively, in one embodiment the chewing gum has a molar ratio between the total amount of active compound according to formula I and the total amount of cyclodextrin which is between 1:10 to 1:1. Preferably, a molar ratio of 1:8-1:1, 1:7-1:1, 1:8-1:2, 1:7-1:2, 1:8-1:3, 1:7:1.3, 1:6-1:3, or 1:6-1:4 exits between the total amount of one or more active compound(s) according to formula I and the total amount of cyclodextrin in the chewing gum according to the invention.

*Amount of complex-bound active compound relative to total amount of compound* In one embodiment, the chewing gum may be one wherein the amount of complex-bound active compound according to formula I is at least 20% by weight of the total amount of a compound according to formula I present in said chewing gum. In preferred embodiments, the amount of complex-bound compound in the chewing gum according to the invention is at least 30% by weight of the total amount of a compound, such as at least 40%, induding at least 50% or even at least 60%.

### Amount of complex-bound compound according to formula I

In useful embodiments, the chewing gum according to the invention is one wherein the complex-bound active compound according to formula I is present in an amount of at least 0.03% by weight of the uncoated chewing gum. In useful embodiments, the complex-bound active compound according to formula I is present in an amount of at least 0.05%, 0.08%, 0.13%, 0.2%, 0.3%, 0.5%, 0.7%, 1%, or 1.25 % by weight of the uncoated chewing gum.

However, the chewing gum comprises in further embodiments the complex-bound compound according to formula I in an amount in the range of 0.03% to 4% by weight of the uncoated chewing gum, such as in the range of 0.8% to 1.25%, including in the range of 0.13% to 1%, e.g. in the range of 0.1% to 2%, such as in the range of 0.2% to 0.7%, including in the range of 0.5% to 1.25%, such as in the range of 0.75% to 2%, including in a range of 0.5% to 4% by weight of the uncoated chewing gum.

The amount present of the complex-bound active compound according to formula I in the chewing gum may also be calculated relative to the gum base. Thus, in useful embodiments, the complex-bound active compound according to formula I is present in an amount of at least 0.01%, 0.02%, 0.08%, 0.13%, 0.2%, 0.5%, 1%, 1.5%, 2%, or 5% by weight of the gum base.

In preferred embodiments, the complex-bound compound according to formula I is present in an amount of 0.01% to 5% by weight of the gum base the inclusion complex is present in the chewing gum according to the invention in an amount in the range of 0.02% to 2% by weight of the gum base, such as in the range of 0.08% to 1.5%, including in the range of 0.13% to 1%, e.g. in the range of 0.2% to 0.5%, such as in the range of 0.5% to 2.5%, including in the range of 1% to 2%, such as in the range of 1.5% to 5%, including in a range of 2% to 5% by weight of the gum base.

### Total amount of active compound according to formula I

The total amount of the active compound according to formula I present in the chewing gum according to the invention may be in an amount of at least 0.03% by weight of the uncoated chewing gum. In useful embodiments, the total amount of the active compound is at least 0.05%, such as at least 0.08%, including at least 0.15%, e.g. at least 0.2%, such as at least 0.3%, including at least 0.5%, e.g. at least 0.7%, such as at least 1%, including at least 1.25 % by weight of the uncoated chewing gum.

However, the chewing gum comprises in further embodiments a total amount of one or more active compound(s) according to formula I which is in the range of 0.03% to 4% by weight of the uncoated chewing gum, such as in the range of 0.08% to 1.25%, including in the range of 0.15% to 1%, e.g. in the range of 0.1% to 1.5%, such as in the range of 0.2% to 0.7%, including in the range of 0.5% to 1.25%, such as in the range of 0.75% to 2%, including in a range of 0.5% to 4% by weight of the uncoated chewing gum.

The total amount of the active compound according to formula I present in the chewing gum may also be calculated relative to the gum base. Thus, in useful embodiments, the total amount of a compound according to formula I present in the chewing gum is at least 0.01, 0.02, 0.08, 0.1, 0.13, 0.5, 1%, 1.5%, 2%, 2.5%, 3%, 3.5%, 4%, 4.5%, 5%, or 5.5% by weight of the gum base.

In preferred embodiments, the total amount of a compound according to formula I present in the chewing gum is in an amount in the range of 0.01% to 5% by weight of the gum base, such as in the range of 0.02% to 2% by weight of the gum base, such as in the range of 0.08% to 1.5%, including in the range of 0.13% to 1%, e.g. in the range of 0.2% to 0.5%, such as in the range of 0.5% to 3%, including in the range of 1% to 2%, such as in the range of 1.5% to 5%, including in a range of 2% to 5% by weight of the gum base.

### Absolute dose of the active compound according to formula I

The active compound according to formula I may be used in the present chewing gum in a dose of 1-30 mg, preferably in the range of 2.0-15 mg, more preferably in the range 2.5-10 mg.

### Amount of complex-bound cyclodextrin relative to total amount of cyclodextrin

As described above, the present chewing gum may comprise free cyclodextrin molecules and/or cyclodextrin molecules which form part of an inclusion complex with one or more active compounds according to formula I, i.e. the cyclodextrin molecules are "complex-bound cyclodextrin".

Accordingly, the chewing gum may be one wherein the amount of complex-bound cyclodextrin is at least 15% by weight of the total amount of cyclodextrin present in said chewing gum. In preferred embodiments, the amount of complex-bound cyclodextrin in the chewing gum according to the invention is at least 20% by weight of the total amount of a compound, such as at least 30%, including at least 40%, e.g. at least 50% or even at least 60%.

### Amount of complex-bound cyclodextrin

In useful embodiments, the complex-bound cyclodextrin is present in an amount of at least 0.1%, 0.15%, 0.4%, 0.6%, 1%, 3%, 5%, 6.5%, 15%, 20%, or 25% by weight of the uncoated chewing gum.

In preferred embodiments, the complex-bound cyclodextrin is present in an amount in the range of 0.1% to 25%, such as in the range of 0.15% to 20%, including in the range of 0.4% to 6.5%, e.g. in the range of 0.6% to 5%, such as in the range of 1% to 3%, including in the range of 1% to 15% by weight of the uncoated chewing gum.

In particularly useful embodiments, the complex-bound cyclodextrin is present in an amount of at least 0.1%, 0.15%, 0.4%, 0.6%, 1%, 3%, 5%, 6.5%, 15%, 20%, or 25% by weight of the gum base.

In further useful embodiments, the complex-bound cyclodextrin is present in an amount in the range of 0.1% to 50% by weight of the gum base, such as in the range of 0.15% to 20%, including in the range of 0.4% to 6.5%, e.g. in the range of 0.6% to 5%, such as in the range of 1% to 3%, including in the range of 1% to 15%, such as in the range of 10% to 30% by weight of the gum base.

### Amount of total cyclodextrin

In useful embodiments, the total amount of cyclodextrin present in the chewing gum is at least 0.03%, 0.15%, 0.4%, 0.6%, 1%, 2%, 2.5%, 5%, 7.5%, 11%, 15%, 20%, 25%, 50% or 70% by weight of the uncoated chewing gum,

However, the total amount of cyclodextrin present may be in an amount in the range of 0.03% to 70%, such as in the range of 1% to 15%, e.g. in the range of 2% to 11%, including in the range of 2.5 % to 7.5%, such as in the range of 5% to 10% by weight of the uncoated chewing gum.

The chewing gum according to the invention may also be one wherein the total amount of cyclodextrin present is an amount of at least 0.1%, 0.15%, 0.4%, 0.6%, 1%, 2%, 2.5%, 5%, 7.5%, 11%, 15%, 20%, 25%, 50% or 70% by weight of the gum base.

In preferred embodiments, the total amount of cyclodextrin present is in an amount in the range of 0.1% to 50%, such as in the range of 1% to 15%, e.g. in the range of 2% to 11%, including in the range of 2.5 % to 7.5%, such as in the range of 5% to 10%, including in the range of 10% to 30% by weight of the gum base.

### Solid state

The inclusion complex useful in the chewing gum of the invention may exist in the form of a hydrate containing varying amounts of water, such as between about 1% and 25% water. The degree of hydration may vary according to, amongst other reasons, the degree of substitution of the hydroxyls, the method of preparation and the molar ratio of the inclusion complex. The water content of the inclusion complex may depend on the manner in which the inclusion complex is stored, the temperature, pressure and relative humidity. Thus, any discussion on the solid state form of the inclusion complex must consider the range of hydrates. The hydrate water is part of the crystal lattice and thus modifying the water content may change the crystal lattice and possibly some of the physical properties of the inclusion complex.

As it is known to the person skilled in the art, cyclodextrin itself forms an inclusion complex with water. Thus, the cyclodextrin used in the preparation of the inclusion complex may be in a hydrated (liquid) form or in an anhydrous form, i.e. in a solid state. However, a skilled person will appreciate that minor amount of water will be present in such an anhydrous form. In a preferred embodiment, the inclusion complex is in a solid state. The presence of an inclusion complex in the solid state to more or less extent can be proved by solid-state analytical methods such as thermoanalytical DSC, X-ray powder diffractometry or solid-phase 13C-NMR spectroscopy. Useful examples of such solid state are, but are not limited to, powder and granulate.

### The chewing gum particles containing gum base

In the chewing gum according to the invention, when having compressed modules comprising gum base, said gum base is typically in the form of compressed gum base particles. The manufacturing of gum base particles is described below. However, the particles may be manufactured according to conventional methods such as those described in the EP 1 474 993, EP 1 474 994 and EP 1 474 995.

It was found, that by using compressed particles of gum base in combination with a specifically defined ratio between the active compound according to formula I and the cyclodextrin, complex-bound in an inclusion complex, as described above, results in an acceptable texture and an interesting taste experience as well as an increased stability of the active compound.

In accordance with the invention, the chewing gum particles comprise gum base. The chewing gum particles may have any form of chewing gum particles containing a certain amount of gum base. The content of gum base in the particles may vary. In some embodiments, the amount of gum base in the chewing gum particles is rather high, such in the range of 40-99% by weight of the chewing gum particles. In some embodiments, the amount of gum base in the chewing gum particles is in the range of 40-90% by weight of the chewing gum particles, such as in the range of 40-80% by weight, including in the range of 40-70% by weight, e.g. in the range of 40-50% by weight, such as in the range of 50-85% by weight, including in the range of 50-75% by weight, e.g. in the range of 50-55% by weight of the chewing gum particles.

In some other embodiments, the amount of gum base in the chewing gum particles is lower, such as in the range of 15-60% by weight of the chewing gum particles. Other useful amounts may vary in the range of 20-60% by weight of the chewing gum particles, such as in the range of 20-60%, including in the range of 20-40% by weight, e.g. in the range of 30-55% by weight, such as in the range of 30-45% by weight of the chewing gum particles. The remaining content of the chewing gum particles may comprise one or more of the below described chewing gum ingredients.

In some embodiments, the particles are made entirely of gum base, substantially without conventional chewing gum ingredients. In this case, the chewing gum ingredients may be applied in the compression process, such as by adding the chewing gum ingredients together with the gum base particles for compression.

In some other embodiments, the particles are made of chewing gum, substantially without further needs for chewing gum ingredients in the compression process. Of course, intermediate solutions may be applicable, such as a varying amount of chewing gum ingredients in the chewing gum particles or in the compression process.

It may be preferred to apply at least a certain amount of high intensity sweetener and/or flavour and/or colour to the chewing gum particles in some embodiments of the invention, such as in case the chewing gum particles substantially consist of gum base.

In preferred embodiments, the average particle size of the particles is in the range of 50-2000 µm measured as the longest dimension of the particle, preferably in the range of 100-1500 µm, and even more preferred in the range of 200-1300 µm.

In even more preferred embodiments, the chewing gum is one wherein at least 70% of the particles have a particle size in the range of 50-2000 µm measured as the longest dimension of the particle, preferably in the range of 100-1500 µm, and even more preferred in the range of 200-1300 µm.

### Gum base

In accordance with the invention, the chewing gum comprises a gum base and at least one inclusion complex comprising a cyclodextrin and one or more active compound(s) according to formula I. The gum base may be used as such or it may be particulated and used as particles containing gum base as described above. Typically, a useful gum base composition comprise one or more elastomeric compounds which may be of synthetic or natural origin, one or more resinous compounds which may be of synthetic or natural origin, fillers, softening compounds and minor amounts of miscellaneous ingredients such as antioxidants and colorants, etc. One advantage of the present invention is that there is no need to adjust the content of other chewing gum ingredients in order to maintain the desired texture. Furthermore, a very interesting observation is that no disintegration of the chewing gum occurs upon chewing.

In addition to the above definition of the expression "gum base", the expression further refers to the water-insoluble part of the chewing gum which typically constitutes 10-99% by weight including the range of 20-99% by weight of the total chewing gum composition, such as the range of 30-99% by weight of the total chewing gum composition. In preferred embodiments, the chewing gum composition comprises gum base in the range of 10-80% by weight of the chewing gum composition, preferably in the range 20-70% by weight, and even more preferably in the range 30-60% by weight of the chewing gum composition.

The chewing gum base, which is admixed with chewing gum ingredients as defined below, can vary substantially depending on the particular product to be prepared and on the desired masticatory and other sensory characteristics of the final product. However, typical ranges (weight %) of the above gum base components are: 5 to 50% by weight elastomeric compounds, 5 to 55% by weight elastomer plasticizers, 0 to 50% by weight filler/texturiser, 5 to 35% by weight softener and 0 to 1% by weight of miscellaneous ingredients such as antioxidants, colorants, etc.

In a preferred embodiment, the gum base of the chewing gum comprises an elastomer. Natural elastomers may include natural rubber such as smoked or liquid latex and guayule as well as natural gums such as jelutong, lechi caspi, massaranduba balata, sorva, perillo, rosindinha, massaranduba chocolate, chicle, nispero, gutta hang kang, and combinations thereof. Useful synthetic elastomers include, but are not limited to, synthetic elastomers listed in U.S. Food and Drug Administration, CFR, Title 21, Section 172,615, the Masticatory Substances, Synthetic, such as polyisobutylene, e.g. having an average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3: 1, polyvinyl acetate (PVA), e.g. having a average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer and combinations hereof.

It is possible to combine a synthetic elastomer having a high molecular weight and a synthetic elastomer having a low molecular weight elastomer in a gum base. Presently preferred combinations of synthetic elastomers include, but are not limited to, polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Typically, the gum base comprises at least one elastomer in an amount in the range of 3-80% by weight of the gum base, preferably in an amount in the range of 4-60% by weight of the gum base, and even more preferred in the range of 5-40% by weight of the gum base, such as in the range of 8-20% by weight of the gum base.

Particularly interesting elastomeric or resinous polymer compounds which advantageously can be used in accordance with the present invention include polymers which, in contrast to currently used elastomers and resins, can be degraded physically, chemically or enzymatically in the environment after use of the chewing gum, thereby giving rise to less environmental pollution than chewing gums based on non-degradable polymers, as the used degradable chewing gum remnants will eventually disintegrate and/or can be removed more readily by physical or chemical means from the site where it has been dumped.

In preferred embodiments, the gum base of the chewing gum according to the invention comprises one or more resins contributing to obtain the desired masticatory properties and acting as plasticizers for the elastomers of the gum base. The resin may be a natural resin and/or it may be a synthetic resin. In the present context, useful resins include, but are not limited to, natural rosin esters, often referred to as ester gums including as examples glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins. Other useful resinous compounds include synthetic resins such as terpene resins derived from alpha-pinene, beta-pinene, and/or d-limonene, natural terpene resins; and any suitable combinations of the foregoing. The choice of resins will vary depending on the specific application, and on the type of elastomer(s) being used.

Usually, the gum base comprises at least one resin in an amount in the range of 10-90% by weight of the gum base, preferably in the range of 20-80% by weight, even more preferred in the range of 30-70% by weight of the gum base, such as in the range of 40-60% by weight of the gum base. In preferred embodiments, the gum base comprises at least one resin in the range of 3-80% by weight of the gum base, preferably in an amount in the range of 4-60% by weight of the gum base, and even more preferred in the range of 5-40% by weight of the gum base, such as in the range of 8-20% by weight of the gum base.

Accordingly, the gum base may furthermore comprise one or more softener. According to the present text, the term "softener" may be used interchangeably with plasticizers and plasticizing agents, and is used for ingredients, which softens the gum or chewing gum formulation and encompass wax, fat, oil, emulsifiers, surfactants, solubilizers etc. The softeners may also include sucrose polyesters, such as glycerin, lecithin, and combinations thereof. Aqueous sweetener solutions such as those containing sorbitol, hydrogenated starch hydrolysates, corn syrup and combinations thereof, may also be used as softeners and binding agents in the chewing gum according to the invention.

In a preferred embodiment, the gum base comprises an emulsifier, which aid in dispersing any immiscible components into a single stable system. The emulsifiers useful in this invention include glyceryl monostearate, lecithin, fatty acid monoglycerides, diglycerides, propylene glycol monostearate, and mixtures thereof. The emulsifier may be employed in an amount in the range of 1-15% by weight of the gum base, and preferably in the range 5-10% by weight of the gum base.

Further examples of useful emulsifier include anionic, cationic, amphoteric or nonionic emulsifiers can be used. Suitable emulsifiers include lecithins, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters ofinteresterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable emulsifiers are polyoxyethylene stearates, such as for instance polyoxyethylene (8) stearate and polyoxyethylene (40) stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllactylate, sodium laurylsulfate, polyoxyethylated hydrogenated castor oil, blockcopolymers of ethylene oxide and propyleneoxide and polyoxyethylene fatty alcohol ether. The emulsifiers may either be a single compound or a combination of several compounds.

Some emulsifier, also referred to as plasticizers, to provide a variety of desirable textures and consistency properties. Because of the low molecular weight of these components, the plasticizers are able to penetrate the fundamental structure of the gum base making it plastic and less viscous. Useful plasticizers include lanolin, palmitic acid, oleic acid, stearic acid, sodium stearate, potassium stearate, glyceryl triacetate, glyceryl lecithin, glyceryl monostearate, propylene glycol monostearate, acetylated monoglyceride, glycerine, and mixtures thereof.

In preferred embodiments, the softener used in the gum base of the chewing gum of the invention is a fat. The fat may e.g. include partially or fully hydrogenated vegetable or animal fats, such as partially or fully hydrogenated coconut oil, partially or fully hydrogenated palm oil, partially or fully hydrogenated palm kernel oil, partially or fully hydrogenated rapeseed oil, partially or fully hydrogenated castor oil, partially or fully hydrogenated maize oil, partially or fully hydrogenated cottonseed oil, partially or fully hydrogenated olive oil, partially or fully hydrogenated sunflower oil, partially or fully hydrogenated safflower oil, partially or fully hydrogenated sesame oil, partially or fully hydrogenated soybean oil, partially or fully hydrogenated beef tallow, and partially or fully hydrogenated lard, and any mixture thereof and any derivative thereof. In useful embodiments, the gum base comprises a fat in an amount in the range of 1-15% by weight of the gum base, and preferably in the range 5-10% by weight of the gum base.

The gum base may furthermore comprise a wax. When a wax is present in the gum base, it softens the polymeric elastomer mixture and improves the elasticity of the gum base. The waxes employed will have a melting point below about 60°C, and preferably between about 45°C and about 55°C. The low melting wax may be a paraffin wax. The wax may be present in the gum base in an amount from about 6% to about 10%, and preferably from about 7% to about 9.5% by weight of the gum base.

In addition to the low melting point waxes, waxes having a higher melting point may be used in the gum base in amounts up to about 5%, by weight of the gum base. Such high melting waxes include beeswax, vegetable wax, candelilla wax, canauba wax, most petroleum waxes, and mixtures thereof.

Further useful waxes include natural and synthetic waxes, hydrogenated vegetable oils, petroleum waxes such as polyurethane waxes, polyethylene waxes, paraffin waxes, microcrystalline waxes, fatty waxes, sorbitan monostearate, tallow, propylene glycol, mixtures thereof, may also be incorporated into the gum base.

Anhydrous glycerin may also be employed as a softening agent, such as the commercially available United States Pharmacopeia (USP) grade. Glycerin is a syrupy liquid with a sweet warm taste and has a sweetness of about 60% of that of cane sugar. Because glycerin is hygroscopic, the anhydrous glycerin may be maintained under anhydrous conditions throughout the preparation of the chewing gum composition.

In an embodiment of the invention, the gum base comprises at least one resin in an amount in the range of 10-90% by weight of the gum base, at least one elastomer in an amount in the range of 4-60% by weight of the gum base, and an emulsifier in an amount in the range of 1-15% by weight. Preferably, the gum base comprises at least one resin in an amount in the range of 30-70% by weight of the gum base, at least one elastomer in an amount in the range of 5-40% by weight of the gum base, and an emulsifier in an amount in the range of 5-10% by weight of the gum base.

In a preferred embodiment, the gum base of the chewing gum according to the invention comprises a filler. The fillers/texturizers may include magnesium and calcium carbonate, sodium sulphate, ground limestone, silicate types such as magnesium and aluminium silicate, kaolin, clay, aluminium oxide, silicium oxide, talc, titanium oxide, mono-, di- and tri-calcium phosphates, cellulose polymers, such as wood, and combinations thereof.

The fillers/texturizers may also include natural organic fibres such as fruit vegetable fibres, grain, rice, cellulose and combinations thereof.

### Chewing gum ingredients

In accordance with the present invention the chewing gum comprises one or more further chewing gum ingredients. Such a chewing gum ingredient may be selected from the group consisting least a bulk sweetener, a high intensity sweetener, a flavouring agent, a colouring agent, a cooling agent, a warming agent, a binding agent, an antioxidant, a pH-regulating agent and an active ingredient.

In a referred embodiment of the present invention, the at least one chewing gum ingredient is a bulk sweetener. The bulk sweetener may be selected from the group consisting of monosaccharides, disaccharides, polysaccharides, sugar alcohols, and mixtures thereof; dextrose, sucrose, lactose, maltose, fructose, xylitol, mannitol, sorbitol, mannitol, maltitol, isomaltol or isomalt, erythritol, lactitol, cyclodextrin randomly bonded glucose polymers such as those polymers distributed under the tradename POLYDEXTROSE^{®} by Pfizer, Inc., Groton, Conn.; isomalt (a racemic mixture of alpha-D-glucopyranosyl-1,6-mannitol and alpha-D-glucopyranosyl-1,6-sorbitol manufactured under the tradename PALATINIT^{®} by Süddeutsche Zucker), maltodextrins; hydrogenated starch hydrolysates; hydrogenated hexoses; and hydrogenated disaccharides.

In an especially preferred embodiment of the invention, the bulk sweetener is present in amount ranging from 10-70% by weight of the chewing gum.

The bulk sweetener may be present in amount ranging from 30-70% by weight of the chewing gum, such as e.g. in the range 35-65% by weight of the chewing gum, and in the range 40-60% by weight of the chewing gum. For example, the bulk sweetener may be present in amount ranging from 20-55% by weight of the chewing gum, such as e.g. in amount ranging from 30-50% by weight of the chewing gum.

The presence of mannitol in the chewing gum surprisingly appears to improve the stability of the active compound according to formula I, relative to other low molecular weight polyol sweeteners such as sorbitol. Thus, in a preferred embodiment of the invention, the bulk sweetener of the chewing gum comprises mannitol in an amount of at least 25% by weight of the total amount of bulk sweetener of the chewing gum, such as in an amount of at least 50% by weight, preferably in an amount of at least 75%, and even more preferred in an amount of at least 95% by weight of the total amount of bulk sweetener of the chewing gum. In an embodiment of the invention, substantially all bulk sweetener of the chewing gum is mannitol.

In another embodiment of the invention, the bulk sweetener of the second compressed module comprises mannitol in an amount of at least 25% by weight of the total amount of bulk sweetener of the second compressed module, such as in an amount of at least 50% by weight, preferably in an amount of at least 75%, and even more preferred in an amount of at least 95% by weight of the total amount of bulk sweetener of the second compressed module.

In an embodiment of the invention, substantially all bulk sweetener of the second compressed module is mannitol.

In interesting embodiment, the chewing gum according to the invention further comprises a high intensity sweetener. Useful high intensity sweetener may be selected from the group consisting of sucralose, neotame, aspartame, salts of acesulfame, alitame, saccharin and its salts, cyclamic acid and its salts, glycyrrhizin, dihydrochalcones e.g. NHDC, thaumatin, monellin, stevioside, Twinsweet (aspartame-acesulfame salt) and combinations thereof.

In order to provide longer lasting sweetness and flavour perception, it may be desirable to encapsulate or otherwise control the release of at least a portion of the artificial sweetener. Likewise, encapsulation may be applied for the purpose of stabilizing the ingredients. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided e.g. using another chewing gum component, such as a resinous compound, as the encapsulation agent.

Usage level of the artificial sweetener will vary considerably depending e.g. on factors such as potency of the sweetener, rate of release, desired sweetness of the product, level and type of flavour used and cost considerations. Thus, the active level of artificial sweetener may vary from about 0.02 to 8% by weight. When carriers used for encapsulation are included, the usage level of the encapsulated sweetener will be proportionally higher. Combinations of sugar and/or non-sugar sweeteners can be used in the chewing gum formulation processed in accordance with the invention. Additionally, the softener may also provide additional sweetness such as with aqueous sugar or alditol solutions.

If a low calorie chewing gum is desired, a low calorie bulking agent can be used. Examples of low calorie bulking agents include polydextrose, Raftilose, Raftilin, Inuline, fructooligosaccharides (NutraFlora^{®}), palatinose oligosaccharided; guar gum hydrolysates (e.g. Sun Fiber^{®}) or indigestible dextrins (e.g. Fibersol^{®}). However, other low calorie-bulking agents can be used.

Flavouring agents may also be useful for the organoleptic properties in the chewing gum according to the invention. The flavouring agents which may be used include those flavouring agents known to the skilled artisan, such as natural and artificial flavouring agents. These flavouring agents may be chosen from synthetic flavour oils and flavouring aromatics and/or oils, oleoresins and extracts derived from plants, leaves, flowers, fruits, and so forth, and combinations thereof. Non-limiting representative flavour oils include spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, and cassia oil. Also useful flavouring agents are artificial, natural and synthetic fruit flavours such as vanilla, and citrus oils including lemon, orange, lime, grapefruit, and fruit essences including apple, pear, peach, grape, strawberry, raspberry, cherry, plum, pineapple, apricot and so forth. These flavouring agents may be used in liquid or solid form and may be used individually or in admixture. Commonly used flavouring agents include mints such as peppermint, menthol, spearmint, artificial vanilla, cinnamon derivatives, and various fruit flavouring agents, whether employed individually or in admixture.

Other useful flavouring agents include aldehydes and esters such as cinnamyl acetate, cinnamaldehyde, citral diethylacetal, dihydrocarvyl acetate, eugenyl formate, p-methylamisol, and so forth may be used. Generally any flavouring agent or food additive such as those described in Chemicals Used in Food Processing, publication 1274, pages 63-258, by the National Academy of Sciences, may be used.

Further examples of aldehyde flavouring agents include, but are not limited to, acetaldehyde (apple), benzaldehyde (cherry, almond), anisic aldehyde (licorice, anise), cinnamic aldehyde (cinnamon), citral, i.e. alpha-citral (lemon, lime), neral, i.e. beta-citral (lemon, lime), decanal (orange, lemon), ethyl vanillin (vanilla, cream), heliotrope, i.e. piperonal (vanilla, cream), vanillin (vanilla, cream), alpha-amyl cinnamaldehyde (spicy fruity flavours), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e. trans-2 (berry fruits), tolyl aldehyde (cherry, almond), veratraldehyde (vanilla), 2,6-dimethyl-5-heptenal, i.e. melonal (melon), 2,6-dimethyloctanal (green fruit), and 2-dodecenal (citrus, mandarin), cherry, grape, strawberry shortcake, and mixtures thereof.

In some embodiments, the flavouring agent may be employed in either liquid form and/or dried form. When employed in the latter form, suitable drying means such as spray drying the oil may be used. Alternatively, the flavouring agent may be absorbed onto water soluble materials, such as cellulose, starch, sugar, maltodextrin, gum arabic and so forth or may be encapsulated. The actual techniques for preparing such dried forms are well-known.

In some embodiments, the flavouring agents may be used in many distinct physical forms well-known in the art to provide an initial burst of flavour and/or a prolonged sensation of flavour. Without being limited thereto, such physical forms include free forms, such as spray dried, powdered, beaded forms, encapsulated forms, and mixtures thereof.

The amount of flavouring agent employed herein may be a matter of preference subject to such factors as the type of final chewing gum, the individual flavour, the gum base employed, and the strength of flavour desired. Thus, the amount of flavouring may be varied in order to obtain the result desired in the final product and such variations are within the capabilities of those skilled in the art without the need for undue experimentation. In chewing gum, the flavouring agent is generally present in amounts from about 0.02% to about 5% by weight, and more specifically from about 0.1% to about 2% by weight, and even more specifically, from about 0.8% to about 1.8%, by weight of the chewing gum.

According to the invention, encapsulated flavours may be added to the final blend and optionally prior to compression. Different methods of encapsulating flavours mixed into the gum base and flavours into the chewing gum may e.g. include spray drying, spray cooling, film coating, coascervation, double emulsion method (extrusion technology) or prilling. Materials to be used for the above-mentioned encapsulation methods may e.g. include gelatine, wheat protein, soya protein, sodium caseinate, caseine, gum arabic, modified starch, hydrolyzed starches (maltodextrines), alginates, pectin, carregeenan, xanthan gum, locus bean gum, chitosan, bees wax, candelilla wax, camauba wax, hydrogenated vegetable oils, zein and/or sucrose.

Useful cooling agents are mentioned in U.S. Patent No. 6,627,233. Particular examples of cooling agents include: menthol, xylitol, menthane, menthone, menthyl acetate, menthyl salicylate, N,2,3-trimethyl-2-isopropyl butanamide (WS-23), substituted p-menthanes, substituted p-menthane-carboxamides (e.g., N-ethyl-p-menthane-3-carboxamide (FEMA 3455)), acyclic carboxamides, substituted cyclohexanamides, substituted cyclohexane carboxamides, substituted ureas and sulphonamides, and substituted menthanols (all from Wilkinson Sword); hydroxymethyl and hydroxyethyl derivatives of p-menthane (from Lever Bros.); menthyl succinate; 2-mercapto-cyclo-decanone (from International Flavors and Fragrances); 2-isopropanyl-5-methylcyclohexanol (from Hisamitsu Pharmaceuticals, hereinafter "isopregol"); hydroxycarboxylic acids with 2-6 carbon atoms; menthone glycerol ketals (FEMA 3807, tradename FRESCOLAT(TM) type MGA); 3-1-menthoxypropane-1,2-diol (from Takasago, FEMA 3784, (hereinafter "TCA")); menthyl lactate; (from Haarman & Reimer, FEMA 3748, tradename FRESCOLAT(TM) type ML). These and other suitable cooling agents are further described in the following U.S. patents: U.S. Pat. Nos. 4,230,688 and 4,032,661 to Rowsell et al.; 4,459,425 to Amano et al.; 4,136,163 to Watson et al.; and 5,266,592 to Grub et al. The cooling agents are typically present in amounts of about 0.001 to about 10% by weight of the chewing gum composition.

Useful warming agents may be selected from a wide variety of compounds known to provide the sensory signal of warming to the user. These compounds offer the perceived sensation of warmth, particularly in the oral cavity, and often enhance the perception of flavours, sweeteners and other organoleptic components. Among the useful warming compounds included are vanillyl alcohol n-butylether (TK-1000) supplied by Takasago Perfumary Company Limited, Tokyo, Japan, vanillyl alcohol n-propylether, vanillyl alcohol isopropylether, vanillyl alcohol isobutylether, vanillyl alcohol n-aminoether, vanillyl alcohol isoamyleather, vanillyl alcohol n-hexyleather, vanillyl alcohol methylether, vanillyl alcohol ethyleather, gingerol, shogaol, paradol, zingerone, capsaicin, dihydrocapsaicin, nordihydrocapsaicin, homocapsaicin, homodihydrocapsaicin, ethanol, isopropol alcohol, iso-amylalcohol, benzyl alcohol, glycerine, and combinations thereof. Furthermore, useful warming agents include capsicum and nicotinate esters, such as benzyl nicotinate.

Colouring agents may be used in amounts effective to produce the desired colour. The colouring agents may include pigments which may be incorporated in amounts up to about 6%, by weight of the chewing gum. For example, titanium dioxide may be incorporated in amounts up to about 2%, and preferably less than about 1%, by weight of the chewing gum. The colourants may also include natural food colours and dyes suitable for food, drug and cosmetic applications. These colourants are known as F.D.& C. dyes and lakes. The materials acceptable for the foregoing uses are preferably water-soluble. Illustrative nonlimiting examples include the indigoid dye known as F.D.& C. Blue No. 2, which is the disodium salt of 5,5-indigotindisulfonic acid. Similarly, the dye known as F.D.& C. Green No. 1 comprises a triphenylmethane dye and is the monosodium salt of 4-[4-(N-ethyl-p-sulfoniumbenzylamino) diphenylmethylene]-[1-(N-ethyl-N-p-- sulfoniumbenzyl)-delta-2,5-cyclohexadieneimine]. A full recitation of all F.D.& C. colourants and their corresponding chemical structures may be found in the Kirk-Othmer Encyclopedia of Chemical Technology, 3rd Edition, in volume 5 at pages 857-884.

In useful embodiments of the invention, the chewing gum comprises an antioxidant, such as lipophilic or hydrophilic antioxidants. Useful lipophilic antioxidants include, but are not limited to, buthylhydroxytoluen (BHT), butylhydroxyanisol (BHA), tocopherols including alpha, beta, gamma and delta (dl-alfa) tocopherols, ascobylpalmitate, tert-butylhydroxyquinone (TBHQ), alkylgallates (e.g. propylgallate), betacarotene, vitamin A, ascorbylpalmitate, ascorbylstearate, phospholipids, and mixtures thereof. Examples of useful hydrophilic antioxidants include, but are not limited to, citric acid, tartaric acid, glycine, ascorbic acid (vitamin C), hydroquinones, sodium ascorbate (vitamin C), calcium ascorbate (vitamin C), salts of sulphur dioxide, including those derived from bisulphite, metabisulphite and sulphite (e.g. sodium sulfite, sodium metabisulphite, potassium-metabisulfit), fumaric acid, malic acid, propionic acid, tartaric acid, phosphoric acid and salts thereof, monothioglycerol, and mixtures thereof.

It will be understood, that it under some circumstances may be useful to use a combination or mixture of two, such as three, four, five or six lipophilic and/or hydrophilic antioxidants.

The antioxidants may be used in the chewing gum according to the invention in an amount in the range of 0.01% to 1% by weight of the uncoated chewing gum, e.g. range of 0.01-0.5%, such as in the range 0.01-0.2%, e.g. in the range 0.01-0.1%,such as in the range of 0.1 - 1%, including in the range of 0.1-0.5%, e.g. in the range 0.1-0.2%, such as in the range 0.1-0.1%, including in the range of 0.2-1%, e.g. in the range 0.5-1% or in the range 0.7-1% by weight of the chewing gum.

Useful pH-regulating agents, acidity regulators, or pH control agents are additives which may be added to the chewing gum to change or maintain pH (acidic, alkaline or pH neutral). They can be organic or mineral acids (acidulants), bases, neutralizing agents, or buffering agents. Examples of useful compounds include ascorbic acid, fumaric acid, adipic acid, lactic acid, malic acid, citric acid, tartaric acid, propionic acid, phosphoric acid and combinations thereof.

Compression adjuvants may also be added. These compounds facilitate compression of the gum into tablets. Suitable compression adjuvants include, but are limited to, glidants, lubricants, wetting agents, diluents, humectants. More specifically, useful compression adjuvants include silicon dioxide, magnesium stearate, calcium stearate, behenic acid, talc and similar substances which can be used to limit the tendency of the gum tablets to stick to the presses.

### Alkalizing agent

Preliminary experiments (not shown here) have indicated that chewing gum according to the present invention gain improved stability by the presence of one or more alkalizing agent. Thus, in a preferred embodiment of the invention, the chewing gum furthermore comprises one or more alkalizing agent(s).

In the context of the present invention, the term "alkalizing agent" covers any compounds which are able to increase the pH of deionized water when added to it.

In an embodiment of the invention, at least one of the one or more alkalizing agent(s) comprises an alkali or alkaline-earth metal hydroxide.

In an embodiment of the invention, at least one of the one or more alkalizing agent(s) comprises a carbonate salt.

In an embodiment of the invention, at least one of the one or more alkalizing agent(s) comprises a bicarbonate salt.

In an embodiment of the invention, at least one of the one or more alkalizing agent(s) comprises a phosphate salt.

In an embodiment of the invention, at least one of the one or more alkalizing agent(s) comprises a borate salt.

In an embodiment of the invention, at least one of the one or more alkalizing agent(s) comprises a basic salt of an organic acid.

In an embodiment of the invention, at least one of the one or more alkalizing agent(s) comprises a basic salt of a carboxylic acid or of a hydroxy carboxylic acid. An example of this is e.g. a basic salt of a food acid. Examples of useful alkalizing agents are a basic salt of ascorbic acid, a basic salt of fumaric acid, a basic salt of adipic acid, a basic salt of lactic acid, a basic salt of malic acid, a basic salt of citric acid, a basic salt of tartaric acid, a basic salt of propionic acid, or combinations thereof.

In an embodiment of the invention, at least one of the one or more alkalizing agent(s) comprises tri-sodium citrate.

The one or more alkalizing agent(s) may be located different places in the chewing gum. In an embodiment of the invention, the first compressed module comprises the one or more alkalizing agent(s).

In a preferred embodiment of the invention, the second compressed module comprises the one or more alkalizing agent(s).

In yet an embodiment of the invention, the first and second compressed modules comprise the one or more alkalizing agent(s), i.e. the one or more alkalizing agent may be present both in first and the second compressed module at the same time.

The stability effect provided by the alkalizing agent appears to be particularly predominant when the one or more alkalizing agent(s) is/are present in the chewing gum an amount sufficient to yield a pH within a specific range as mentioned below when the chewing gum is submerged and partially dissolved in water.

Thus, in a preferred embodiment of the invention, the one or more alkalizing agent(s) is/are present in the chewing gum an amount sufficient to form a pH in the range of pH 5-12, preferably in the range of pH 5.5-11, and even more preferably in the range of pH 6-10, such as in the range of pH 6.5-9, or in the range of pH 7-9,
wherein the formed pH is determined by submerging the chewing gum chewing gum chewing gum in 50 mL deionized water, stirring the mixture of the chewing gum and deionized water for 30 minutes, and then immediately measuring the pH of the mixture, and wherein the temperature of the mixture is maintained at approx. 25 degrees C during the stirring and the measurement.

In embodiments where the chewing gum comprises a water-insoluble coating, the pH determination is performed using the uncoated chewing gum.

In another embodiment of the invention, the one or more alkalizing agent(s) is/are present in the second compressed module in an amount sufficient to form a pH in the range of pH 5-12, preferably in the range of pH 5.5-11, and even more preferably in the range of pH 6-10, such as in the range of pH 6.5-9, or in the range of pH 7-9,
wherein the formed pH is determined by submerging the second compressed module in 50 mL deionized water, stirring the mixture of the second compressed module and deionized water for 30 minutes, and then immediately measuring the pH of the mixture, and wherein the temperature of the mixture is maintained at approx. 25 degrees C during the stirring and the measurement.

The amount of the one or more alkalizing agent(s) varies with the selection of the active compound according to formula I and the other excipients of the chewing gum. In an embodiment of the invention, the chewing gum comprises the one or more alkalizing agent(s) in an amount in the range of 0.01-25% by weight of the chewing gum, preferably in the range of 0.1-10% by weight of the chewing gum, and even more preferred in the range of 0.25-5% by weight of the chewing gum.

In yet an embodiment of the invention, the first compressed layer comprises the one or more alkalizing agent(s) in an amount in the range of 0.01-25% by weight of the first compressed layer, preferably in the range of 0.1-10% by weight of the first compressed layer, and even more preferred in the range of 0.25-5% by weight of the first compressed layer.

In preferred embodiment of the invention, the second compressed layer comprises the one or more alkalizing agent(s) in an amount in the range of 0.01-25% by weight of the second compressed layer, preferably in the range of 0.1-10% by weight of the second compressed layer, and even more preferred in the range of 0.25-5% by weight of the second compressed layer.

While the one or more alkalizing agent(s) may be present in the chewing gum in many different forms, it is presently preferred that at least one of the one or more alkalizing agents(s) is in particulate form.

In another embodiment of the invention, at least one of the one or more alkalizing agents(s) is in intimate contact with the active compound according to formula I. Intimate contact may e.g. be accomplished by granulated the at least one of the one or more alkalizing agents(s) with the active compound according to formula I. Alternatively it may be accomplished by pre-blending the at least one of the one or more alkalizing agents(s) with the active compound according to formula I with the active compound according to formula I before the preparing the powder mixture for compression. Pre-blending is particularly effective if the average particle size of the alkalizing agent is substantially smaller than the particle size of the particles comprising the active compound according to formula I.

### Tablet material

In accordance with the present invention, the second and/or third compressed module of the chewing gum may comprise tablet material. The expression "tablet material" is in the present context used for the above described chewing gum ingredients when these are used in a compressed module comprising tablet material. However, examples of further useful tablet materials include, but are not limited to, conventional pharmaceutical acceptable excipients such as a glidant, a lubricant, a filler substance, and a dry or wet binder.

Examples of useful glidants and lubricants are stearic acid, metallic stearates, talc, colloidal silica, sodium stearyl fumarate and alkyl sulphates.

In the present invention, a dry binder such as e.g. sorbitol, isomalt, or mixtures thereof may be used. The dry binder provides the effect of binding a material and thereby providing a powder that can be compressed into a tablet.

A wet binder is an excipient that in combination with water facilitates a powder to be compressed into tablets. A wet binder must, at least to some extent, be soluble in water. Examples of wet binders are PVP (polyvinylpyrrolidone), HPMC (hydroxymethylpropyl-cellulose) or gelatine.

A filler substance may be any pharmaceutically acceptable substance that does not interact with the active compound according to formula I or with other excipients. Useful filler substances include mannitol, dextrins, maltodextrins, inositol, erythritol, isomalt, lactitol, maltitol, mannitol, xylitol, sorbitol, low-substituted hydroxypropylcellulose, starches or modified starches (e.g potato starch, maize starch, rice starch, pre-gelatinised starch), polyvinylpyrrolidone, polyvinylpyrrolidone/vinyl acetate copolymer, agar (e.g. sodium alginate), carboxyalkylcellulose, dextrates, gelatine, gummi arabicum, hydroxypropyl cellulose, hydroxypropylmethylcellulose, methylcellulose, microcrystalline cellulose polyethylene glycol, polyethylene oxide, polysaccharides e.g. dextran, soy polysaccharide, sodium carbonate, and sodium chloride.

### Coating

In accordance with the invention, the chewing gum may comprise a coating applied onto the chewing gum centre. In the present context, a suitable coating is any coating that results in extended storage stability of the chewing gum products as defined above, relative to a chewing gum of the same composition that is not coated. Thus, suitable coating types include hard coatings, soft coatings, film coatings and sealing coatings of any composition including those currently used in coating of chewing gum, pharmaceutical products and confectioneries.

The chewing gum comprises the coating in an amount in the range of 1-80% by weight of the chewing gum, such as in an amount in the range of 10-50%, or 15-45% by weight of the chewing gum. Preferably, the chewing gum comprises the coating in an amount in the range of 20-40% by weight of the chewing gum.

In a useful embodiment of the invention, the coating comprises an inclusion complex comprising cyclodextrin and one or more active compound(s) according to formula I. The coating may e.g. comprise the inclusion complex in an amount in the range of 1-30 mg, and preferably in the range of 5-20 mg. Preferably, the coating comprises the inclusion complex in an amount in the range of 1-10% by weight of the coating.

The coating may be a hard coating, which term is used in the conventional meaning of that term including sugar coatings and sugar-free (or sugarless) coatings and combinations thereof. The objects of hard coating are to obtain a sweet, crunchy layer, which is appreciated by the consumer, and to protect the composition for various reasons. In a typical process of providing the composition with a protective sugar coating the gum centers are successively treated in suitable coating equipment with aqueous solutions of crystallizable sugar such as sucrose or dextrose, which, depending on the stage of coating reached, may contain other functional ingredients, e.g. fillers, colours, etc. In the present context, the sugar coating may contain further functional or active compounds including flavour compounds, pharmaceutically active compounds and/or polymer degrading substances.

In the production of chewing gums it may, however, be preferred to replace the cariogenic sugar compounds in the coating by other, preferably crystallizable, sweetening compounds that do not have a cariogenic effect. In the art such coating is generally referred to as sugarless or sugar-free coatings. Presently preferred non-cariogenic hard coating substances include polyols, e.g. sorbitol, maltitol, mannitol, xylitol, erythritol, lactitol, isomalt and tagatose which are obtained by industrial methods by hydrogenation of D-glucose, maltose, fructose or levulose, xylose, erythrose, lactose, isomaltulose and D-galactose, respectively. One advantage of using polyols in the coating is that they act simultaneously as a sweetener and as an masking agent for the bitter taste of one or more active compound(s) according to formula I.

In a typical hard coating process, a syrup containing crystallizable sugar and/or polyol is applied onto the chewing gum and the water it contains is evaporated off by blowing with warm, dry air. This cycle may be repeated several times, typically 10 to 80 times, in order to reach the swelling required. The term "swelling" refers to the increase in weight of the products, as considered at the end of the coating operation by comparison with the beginning, and in relation to the final weight of the chewing gum.

A sealing coating of e.g. shellac, ethyl cellulose, zein, acrylic compounds or carnauba wax may be applied over the hard coating, if desired, in order to seal the crunchy coating to reduce the exposure of the coating to atmospheric moisture.

Alternatively, the coating may be a soft coating. Such a soft coating is applied using conventional methods and may advantageously consist of a composition of a sugar or any of the above non-cariogenic, sugar-less sweetening compounds and a starch hydrolysate.

In a preferred embodiment of the invention, the chewing gum tablet comprises a film coating. The film coating may be obtained by subjecting the composition to a film coating process and which therefore comprises one or more film-forming polymeric agents and optionally one or more auxiliary compounds, e.g. plasticizers, pigments and opacifiers. A film coating is a thin polymer-based coating applied to a composition of any of the above forms. The thickness of such a film coating is usually between 20 and 100 µm. Generally, the film coating is obtained by passing the composition through a spray zone with atomized droplets of the coating materials in a suitable aqueous or organic solvent vehicle, after which the material adhering to the composition is dried before the next module of coating is received. This cycle is repeated until the coating is complete.

In the present context, suitable film-coating polymers include edible cellulose derivatives such as cellulose ethers including methylcellulose (MC), hydroxyethyl cellulose (HEC), hydroxypropyl cellulose (HPC) and hydroxypropyl methylcellulose (HPMC). Other useful film-coating agents are acrylic polymers and copolymers, e.g. methylacrylate aminoester copolymer or mixtures of cellulose derivatives and acrylic polymers. Useful polymers may include: cellulose acetate phtalate (CAP), polyvinyl acetate phtalate (PVAP), shellac, metacrylic acid copolymers, cellulose acetate trimellitate (CAT) and HPMC. It will be appreciated that the outer film coating according to the present invention may comprise any combination of the above film-coating polymers.

In other embodiments of the invention, the film-coating layer of the chewing gum tablet comprise a plasticizing agent having the capacity to alter the physical properties of a polymer to render it more useful in performing its function as a film forming material. In general, the effect of plasticizers will be to make the polymer softer and more pliable as the plasticizer molecules interpose themselves between the individual polymer strands thus breaking down polymer-polymer interactions. Most plasticizers used in film coating are either amorphous or have very little crystallinity.

In the present context, suitable plasticizers include polyols such as glycerol, propylene glycol, polyethylene glycol, e.g. the 200-6000 grades hereof, organic esters such as phtalate esters, dibutyl sebacate, citrate esters and triacetin, oils/glycerides including castor oil, acetylated monoglycerides and fractionated coconut oil.

The choice of film-forming polymer (s) and plasticizing agent (s) for the film coating of the composition is made with due consideration for achieving the best possible barrier properties of the coating in respect of dissolution and diffusion across the film of moisture and gasses.

The film coating of the chewing gum tablet may also contain one or more colorants or opacifiers. In addition to providing a desired colour hue, such agents may contribute to protecting the compressed gum base against pre-chewing reactions, in particular by forming a barrier against moisture and gasses. Suitable colorants/opacifiers include organic dyes and their lakes, inorganic colouring agents, e.g. titanium oxide and natural colours such as e.g. beta-carotene.

Additionally, film coatings may contain one or several auxiliary substances such as flavours and waxes or saccharide compounds such as polydextrose, dextrins including maltodextrin, lactose, modified starch, a protein such as gelatine or zein, a vegetable gum and any combination thereof.

The coating, in general, typically comprises one or more layers. For example the number of layers of the coating may be in the range of 1-100 layers, such as 3-75 layers, 10-60 layers, and 20-40 layers.

The coating comprises for example a wax layer. In an embodiment of the invention, the outermost layer of the coating is a wax layer.

A chewing gum according to the present invention, has typically a weight in the range of 0.1-10 g, such as in the range of 0.5-5 g or in the range of 0.75-2.5 g, preferably in the range of 0.8-2 g, and even more preferred in the range of 1-1.5 g. Compressed chewing gums according to the invention, have typically a weight in the range of 0.5-3.0 g, such as in the range of 0.75-2.5 g or in the range of 0.8-2.0 g, preferably in the range of 1.0-1.5 g. Center-filled chewing gums normally have weights in the range of 0.5-5 g, preferably in the range of 1-4 g, and even more preferred in the range of 2-3 g. Typical weights for bead shaped chewing gums are in the range of 0.1-0.6 g, preferably in the range of 0.2-0.5 g, and even more preferred in the range of 0.3-0.4 g.

It should be noted that, according to the present invention, embodiments and features described in the context of one of the aspects of the present invention also apply to the other aspects of the invention.

### Method of preparing a chewing gum comprising an inclusion complex

In accordance with the present invention there are provided several methods for preparing a chewing gum comprising an inclusion complex comprising a cyclodextrin and one or more active compound(s) according to formula I. In general, medicated chewing gums are prepared by sequentially adding the various chewing gum ingredients to a commercially available kneading kettles or mixer known in the art in order to produce a homogeneous chewing gum composition.

The kneading kettles are heated to a temperature of 30-80°C, typically approx. 50-60°C. The mixing process starts with mixing and melting the gum base for 1-20 min, typically approx. 10 minutes. The gum base may also be melted before adding. All or a part of the bulk sweetener(s) may be added and mixed at the same time together with the gum base.

Subsequent, the sweeteners and further ingredients are added and following mixing for further 1-20 minutes, typically approx. 5-10 minutes.

Adding and mixing of the inclusion complex may be at the same time as the sweeteners and further ingredients or added separately in the following step and mixed for further 10-30 min., typically approx. 20 min.

Flavours are added together or separately in additional steps and mixed for further 1-15 minutes, typically 5-10 minutes. The adding and mixing of flavours may also take place in the beginning of the mixing process, i.e. before the admixture of sweetener and further ingredients. It is also possible to add flavours in two or more portions during the mixing process.

Total mixing time to produce a homogeneous gum mass with the chewing gum components may take from 14 to 95 minutes, typically 45 to 55 minutes.

When mixing is completed, the mixer is tipped, and the gum mass is taken out into rolling bins, carts, onto trays.

Before forming the chewing gum core, the chewing gum mass must be cooled until the right texture of the gum mass is achieved. The temperature of the gum mass is 30-80°C, typically 50-65°C. In order to form the chewing gum cores, the temperature of the gum mass must be reduced to 30-50 °C by cooling at room temperature or in a cooled place/room (controlled temperature and moisture) or through a cooling tunnel to achieve a quicker cooling of the gum mass.

The forming of the chewing gum cores may take place by extrusion through a specially formed nozzle, or after extrusion by means of rollers, punching machines, tentering wheels. The chewing gum core may be formed into cores suitable shape, e.g. rectangular pellets/tablets, sticks, balls, cubes, cylinders, and many other shapes.

In order to prevent the chewing gum cores from sticking to rollers or other tools, the chewing gum mass is frequently powdered with e.g. icing sugar, talc, corn flour.

The formed chewing gum cores can be hardened and cooled to room temperature in a cooling tunnel or the cooling may take place on trays at a store room for semi-manufactured products at a controlled temperature and moisture condition.

The formed and cooled chewing gum cores may be coated and polished before the packing. The coating of the chewing gum cores may take place in a tilted, round, horizontally placed cylindrical coating kettle that rotate during the coating and polishing process. The coating kettle may be made from copper, stainless steel or fiberglass-reinforced polyester, and are often equipped with piping systems that supply and exhaust air and dose the coating suspension, powder sweetener, flavours, wax, talc etc. Coaters with perforated drum like the Dria-coater or Fluid bed coaters may also be used for coating of the product

The coating suspension is produced and heated up to 75°C depending on the sweetener; when xylitol is used, the suspension is heated up to about 70 °C.

Cores of chewing gum are first separated in the rotating coating kettles. The coating suspension with xylitol is added in small portions to the kettle and dispersed evenly over the surfaces of the cores after some time to smooth out. Powder may be added in connection with each or some of the suspension portions. The operation is repeated until the specified weight has reached typically about 1.1 g to 1.2 g. Dose and smooth the surfaces of the tablets with xylitol suspension until the tablet weight is 1.3 g. The coat weight may be up to 500 mg, but typically 200 to 300 mg is preferred.

Flavour(s) might be added during coating of the chewing gum product, either together with a small dosage of the coating suspension or by it self.

High potency sweetener(s) may also be added in the coat, either added in the formulation of the produced coating suspension or together with a small dose the coating suspension or by it self. Preferably the high potency sweeteners are added in the formulation of the coating suspension to achieve a more homogeneous distribution of the sweeteners.

In order to achieve a shinning surface of the chewing gum product, it may subsequently be subjected to a polishing. The polishing may also take place in rotating kettles in which a polishing suspension or polishing powder is added to the coated cores in one or more portion(s). The polishing suspension often consists of wax, emulsifier, coating agent, gum arabic, water, etc. The polishing powder often consists of wax only, or of wax mixed with emulsifiers, gum Arabic or talc, etc.

Accordingly, in an aspect of the present invention, there is provided a method of preparing a chewing gum comprising at least one inclusion complex comprising a cyclodextrin and one or more active compound(s) according to formula I, the method comprising the steps of a) providing at least one inclusion complex comprising a cyclodextrin and a compound according to formula I; b) providing a gum base; c) optionally providing one or more further chewing gum ingredients, d) mixing said inclusion complex and gum base, and optionally the one or more further chewing gum ingredients, and thus obtaining a mixture; and e) shaping the mixture to obtain the chewing gum.

In a useful embodiment, the inclusion complex comprising a cyclodextrin and a compound according to formula I is in a solid state, such as a powder or as a granulate, as described above.

It will be understood, that the further chewing gum ingredients may be selected from the above group consisting of a bulk sweetener, a high intensity sweetener, a flavouring agent, a colouring agent, a cooling agent, a warming agent, a binding agent, an antioxidant, a pH-regulating agent and an active ingredient.

In further aspects, there are provided methods for preparing chewing gums comprising compressed modules, such as one or multiple compressed modules. Initially, chewing gum particles containing gum base are provided.

The chewing gum particles may be in any suitable form according to the invention. As described above, in some embodiments, the particles have been particulated prior to application. Particulation may be in any form of "building up" particles from smaller primary particles into macro particles or in any form of "building down" from larger substances into macro particles. Any form of particulation may be applied, such as granulation, pelletizing, agglomeration, or any other suitable means for particulation.

Granulation may be applied in some embodiments as a means for particulation, resulting in granules. Granules should be understood in its broadest content. In some embodiments of the invention, the granules may be a result of a total chewing gum manufacture, where the chewing gum after production is comminuted into smaller particles, optionally under cooling conditions such as with a coolant or physical cooling, where after these particles are pressed together, optionally using at least some further processing aids. The comminuted particles may be achieved by grinding, milling, or any other suitable processing means.

Thus, in a specific embodiment the chewing gum particles are provided by a method where the particles are obtained through grinding of the prepared chewing gum composition. More specifically, such a method comprises the steps of a) mixing of a soft basic gum base with at least one sweetener and, optionally, at least one other chewing gum ingredient, at a temperature of between 35 and 75°C; b) cooling of the mixture thus obtained to a temperature of between 0 and -40°C and, preferably, between -10 and -40°C; c) grinding and subsequent screening of the mixture thus obtained to a particle size of less than 10 mesh; and d) optional mixing of the powder thus obtained with at least one anti-agglutination agent.

Agglomeration may also be applied in some other embodiments as a means for particulation, resulting in agglomerates.

Pelletizing may be applied in some other embodiments as a means for particulation, resulting in pellets. The pellets may be partly manufactured as a result of an extruding process. In some embodiments, the pellets are pelletized in an underwater process, whereby gum base are pressed through dies in a die plate, meaning openings of a certain diameter, into a cooling media and thereupon dried. In some other embodiments, the pellets are pelletized in a strand pelletizing process with cool air.

Thus, in a specific embodiment, the chewing gum particles containing gum base are provided by a method comprising at least the steps of a) feeding a gum base into an extruder; b) pressurizing the gum base in the extruder; c) extruding the gum base through a die means; and d) cutting the extruded gum base in a liquid filled chamber.

In useful embodiments, the provided chewing gum particles are made entirely of a gum base, substantially without conventional chewing gum ingredients. In this case, the chewing gum ingredients may be applied in the compression process, such as by adding the chewing gum ingredients together with the gum base particles for compression.

However, under some circumstances it may be useful to provide chewing gum particles made entirely of a chewing gum composition, substantially without further needs for chewing gum ingredients in the compression process.

Chewing gum ingredients, e.g. flavours and sweeteners, may with advantage be added to the gum base in order to obtain a composition in the extruder immediately before the composition is extruded through the die means into the water filled chamber where the extruded and cut chewing gum composition is immediately cooled to low temperatures.

Of course, intermediate solutions may be applicable, such as a varying amount of chewing gum ingredients in the chewing gum particles or in the compression process. It may be preferred to apply at least a certain amount of high intensity sweetener and/or flavour and/or colour to the chewing gum particles in some embodiments of the invention, such as in case the chewing gum particles substantially consist of gum base.

By adding the chewing gum ingredients to the chewing gum particles, the ingredients are only subjected to elevated temperatures during the extrusion, such as only during the latter part thereof, and the short duration of the extrusion and the quick cooling in the water prevents or reduces decomposition of fragile flavours components, and thus preserving a maximum of the components. This is especially important for natural flavours in order to maintain the full natural taste of the flavour.

In one embodiment of the present invention, the chewing gum is a chewing gum comprising one or more compressed module(s), i.e. a compressed chewing gum. The compression is preferably performed by applying pressure to the mixture of chewing gum particles, ingredients etc., whereby the bulk volume is reduced and the amount of air is decreased. During this process energy is consumed. As the components of the mixture come into closer proximity to each other during the volume reduction process, bonds may be established between the components. The formation of bonds is associated with a reduction in the energy of the system as energy is released. Volume reduction takes place by various mechanisms and different types of bonds may be established between the components depending on the pressure applied and the properties of the components.

In one aspect of the present invention, there is provided a method of preparing a chewing gum comprising at least one inclusion complex comprising a cyclodextrin and one or more active compound(s) according to formula I having one or a first compressed module, the method comprising the steps of: a) providing a portion comprising at least one inclusion complex comprising a cyclodextrin and one or more active compound(s) according to formula I, and chewing gum particles containing gum base; b) optionally providing a portion of one or more further chewing gum ingredients; c) dosing the portion comprising the at least one inclusion complex comprising a cyclodextrin and one or more active compound(s) according to formula I, and the chewing gum particles containing gum base, and optionally the one or more further chewing gum ingredients; and d) compressing a) and b) after dosing, to obtain a first compressed module.

Thus, the chewing gum is prepared by providing a portion comprising an inclusion complex comprising a cyclodextrin and one or more active compound(s) according to formula I, and chewing gum particles containing gum base. Subsequent, the portions are individually dosed, i.e. the portions are individually loaded in the tablet machine, and compressed together under high pressure (typically when applying cooling) into a compressed module. Any tablet pressing machine may be used which is capable of pressing tablets comprising particles containing chewing gum base.

In one aspect of the present invention, there is provided a method of preparing a chewing gum comprising at least one inclusion complex comprising a cyclodextrin and one or more active compound(s) according to formula I having one or a first compressed module, the method comprising the steps of a) providing a portion comprising at least one inclusion complex comprising a cyclodextrin and a compound according to formula I, and chewing gum particles containing gum base; b) optionally providing one or more further chewing gum ingredients; c) mixing the portion comprising at least one inclusion complex comprising a cyclodextrin and a compound according to formula I, and the chewing gum particles containing gum base, and optionally the one or more further chewing gum ingredients, thus obtaining a mixture; and d) compressing the mixture, to obtain a first compressed module.

In accordance with the present invention, one or more chewing gum ingredients may, as described above, be provided and compressed together in step d) with the portion comprising the inclusion complex and the chewing gum particles containing gum base. However, the one and more chewing gum ingredients may also be added to the gum base in the extruder as described above.

In a useful embodiment, the method according to the invention furthermore comprises a step of coating the first compressed module with the above mentioned coatings.

In an embodiment, the method furthermore comprises the steps of e) compressing a portion comprising tablet material to obtain a second compressed module; f) contacting the first compressed module with the second compressed module, to obtain a coherent compressed chewing gum tablet comprising a first and a second compressed module.

Useful tablet materials are mentioned above. Furthermore, the method comprises a step of coating the coherent compressed chewing gum tablet.

A further aspect relates to a method of preparing a chewing gum comprising at least one inclusion complex comprising a cyclodextrin and one or more active compound(s) according to formula I having two compressed modules, the method comprising the steps a) providing chewing gum particles containing gum base and optionally portion(s) comprising one or more chewing gum ingredients; b) providing a portion comprising tablet material and a portion comprising at least one inclusion complex comprising a cyclodextrin and one or more active compound(s) according to formula I; c) compressing a), to obtain a first compressed module; d) contacting the first compressed module with b); e) compressing b) and the first compressed module, to obtain a coherent compressed chewing gum comprising a first compressed module and a second compressed module.

A further step of the present method according to the invention comprises a step of coating the coherent compressed chewing gum tablet of step e).

In a further aspect, there is provided a method of preparing a compressed chewing gum comprising at least one inclusion complex comprising a cyclodextrin and one or more active compound(s) according to formula I having three compressed modules, the method comprising the steps of a) providing chewing gum particles containing gum base, a portion comprising at least one inclusion complex comprising a cyclodextrin and a compound according to formula I, and optionally portion(s) comprising one or more chewing gum ingredients; b) providing a portion comprising tablet material and optionally a portion comprising at least one inclusion complex comprising a cyclodextrin and a compound according to formula I; c) providing a portion comprising tablet material and a portion comprising at least one inclusion complex comprising a cyclodextrin and a compound according to formula I; d) locating b) and c) on opposite sites of a) following a sequence of one or more compressing step(s), to obtain a coherent compressed chewing gum tablet comprising a first compressed module and a second compressed module and a third compressed module.

A further step of the present method according to the invention comprises a step of coating the coherent compressed chewing gum tablet of step d).

In preferred embodiments of all above methods for preparing a medicated chewing gum, further ingredient are added selected from the group consisting of a bulk sweetener, a high intensity sweetener, a flavouring agent, a colouring agent, a cooling agent, a warming agent, a binding agent, an antioxidant, a pH-regulating agent and an active ingredient.

In useful embodiments of all above methods for preparing a medicated chewing gum, the method comprising a step prior to step a) wherein the compound(s) according to formula I is complex bound to cyclodextrin in order to obtain an inclusion complex comprising the compound(s) according to formula I and cyclodextrin.

The present inventors found, that during the preparation of the inclusion complex a hydroxyl acid salt, such as sodium citrate, or carboxymethylcellulose may be added during the complex binding. Without being bound by theory, it is believed that the addition of a hydroxyl acid salt or carboxymethylcellulose to the suspension results in increased suspension homogeneity, and thus can be used as a suspension improving additive in the present invention. Further useful additives for improving the homogeneity of the suspension comprising active compounds according to formula I and cyclodextrin include, but are not limited hereto, sodium hydroxide, potassium-sodium-tartarate (K-Na-tartarate), tartaric acid and citric acid.

Subsequently, the obtained solution of an inclusion complex may be dried by freeze-drying or spray-drying in order to obtain a powder or granulate of the inclusion complex.

Yet an aspect of the invention relates to an oral dosage form identical to the chewing gum as defined herein, but with the one exception that all gum base has been replaced with an inert excipient, such as bulk sweetener. All aspects and embodiments mentioned herein, except for details relating to the gum base, also apply to the oral dosage form.

The oral dosage form may be in the form of a capsule; a tablet, and particularly an effervescent tablet or a fast disintegrating tablet; a liquid syrup; a dry syrup; a lozenge; or a hardboiled confectionery. In a preferred embodiment of the invention, the oral dosage form is in the form of a tablet.

### Use of an inclusion complex

An interesting aspect of the present invention relates to a method for improving the stability of one or more active compound(s) according to formula I contained in a medicated chewing gum, comprising the steps of complex binding said compound(s) to a cyclodextrin.

A further aspect of the present invention relates to the use of cyclodextrin to protect one or more active compound(s) according to formula I against degradation, such as against oxidation in a chewing gum, wherein said cyclodextrin forms an inclusion complex with said compound(s).

Furthermore, the invention relates to the use of an inclusion complex comprising a cyclodextrin and one or more active compound(s) according to formula I in a mediated chewing gum comprising the compound(s) according to formula I for improving the stability of said compound(s).

In preferred embodiments, the stability of said compound is improved by at least 4%, such as at least 5%, preferably at least 10%, more preferably at least 15% when stored for 1 month at a temperature of 40°C and a humidity of 75% RH compared to a mediated chewing gum comprising one or more active compound(s) according to formula I not complex-bound to a cyclodextrin stored under the same conditions.

A final aspect of the present invention relates to the use of a cyclodextrin in a chewing gum comprising one or more active compound(s) according to formula I, wherein at least 80%, such as at least 85%, preferably at least 90%, more preferably at least 95%, most preferably 99% of the initial amount of said compound(s) is left after storage for 1 month, 2 months or even 3 months at a temperature of 40°C and a humidity of 75% RH, wherein said cyclodextrin forms an inclusion complex with said compound(s).

The invention will now be described in further details in the following non-limiting examples.

### EXAMPLES

### EXAMPLE 1

### Preparation of an inclusion complex comprising a cyclodextrin and cetirizine

### 1.1 Material and Methods

The following cyclodextrin were used:
- α-cyclodextrin
- β-cyclodextrin
- γ-cyclodextrin

Approximately 1:5 mole/mole Cetirizine.2HCl and cyclodextrin was mixed intensively in the presence of water in a glass reactor at room temperature with a magnetic stirrer. The detailed process is as follows:
- 100 g of cetirizine-2HCl was dissolved in 300 ml of deionised water by intense agitation at room temperature. The resulting solution was filtered across a membrane of 0.45 µm pore size to remove remaining active charcoal;
- 1500 g of cyclodextrin hydrate (12% water content) was transferred into a 5 litres volume glass reactor equipped with stirring;
- 2.7 litres of deionised purified water was added to the cyclodextrin and the mixture was stirred intensively with 600 r.p.m;
- Calculated molar ratios of 1:1, 1:2 and 1:4 of cetirizine-2HCl and cyclodextrin were reacted in the presence of water;
- The previous prepared 300 ml cetirizine-2HCl solution (containing 100g cetirizine-2HCl) was added to the stirred cyclodextrin suspension and the cyclodextrin-cetirizine-2HCl mixture was stirred for four hours at 600 r.p.m. at an ambient temperature.

The well-homogenized mixture was dried using two different drying methods: freeze-drying and spray-drying.

*Freeze-drying:* The reaction mixture was stirred with 600 r.p.m. at room temperature for 4 hours followed by chilling to minus 55-60 °C on a mixture of dry ice and ethanol. Water was removed from the suspension by freeze-drying.

*Spray-drying:* Immediately after stirring the homogenized reaction mixture was transferred into the spray-drier (it was important to maintain the non-sedimented state of the reaction mixture). Subsequent, the suspension was spray-dried with an input temperature of 200°C, an output temperature of 95°C and a head rotation of the spray-drier of 22.000 rotations per minute. The complete spray-drying time was 35 minutes.

### 1.2 Conclusion

The following conclusions were made:
- β-cyclodextrin was shown to be the most feasible cyclodextrin for developing a potential cetirizine-containing product;
- The result of the study indicate that a cetirizine.2HCl/ β-cyclodextrin formulation should contain around 7% active cetirizine and around 5% residual water by weight;
- Spray-drying is the preferred drying method.

### EXAMPLE 2

### Improvement of suspension homogeneity in the cetirizine.2HCL/β-cyclodextrin reaction mixture by non-complex forming additives

The homogeneity of the suspension used for the production of an inclusion complex comprising cetirizine and β-cyclodextrin is an important factor of the spray drying technology. Different polymers are tested her for use as additives in order to get more homogeneous suspensions.

### 2.1 Materials and methods

### Standard preparation

Different amounts of β-cyclodextrin (40 - 60 mg) are accurately weighed into a 5 ml graduated flask and dissolved in water and filled up to the mark with the same solvent.

**Table 2.1 Tested non-complex forming additives**

| **Abbreviation** | **Substance** |
|---|---|
| NaOH | Sodium hydroxide |
| K-Na-tartarate | Potassium-sodium-tartarate |
| Tartaric acid | Tartaric acid |
| Citric acid | Citric acid |
| Na-citrate | Tri-sodium citrate |
| HPC | Hydroxypropyl cellulose |
| HPMC | Hydroxypropyl-methyl cellulose |
| CMC | Carboxymethyl cellulose |
| PVP | Polyvinylpirrolidone |

### Sample preparation

About 50 mg of cetirizine.2HCL/β-cyclodextrin preformulation sample are accurately weighed into a 5 ml graduated flask and dissolved in water and filled up to the mark with the same solvent.

### HPLC method

The effect of the additives on the stability of suspension, namely the β-cyclodextrin content of filtrates of cetirizine.2HCL/β-cyclodextrin reaction mixture, was visually studied using a HPLC method using a CD-Screen-I HPLC column (particle size: 5µm, column length: 250 mm, inner diameter: 4.0 mm). The mobile phase was for channel A: 1000 ml water containing 2 ml formic acid; and channel B: 900 ml acetonitrile, 100 ml water and 2 ml trifluoroacetic acid. The following solvent gradient program was applied to eluate β-cyclodextrin and cetirizine from the column:

| Time [min.] | Channel A [%] | Channel B [%] |
|---|---|---|
| 0 | 100 | 0 |
| 3 | 100 | 0 |
| 20 | 40 | 60 |

### Stop time: 20 min., Post time: 10 min.

| | |
|---|---|
| Flow rate: | 1.0 ml/min. |
| Column temperature: | 40 °C |
| Injection volume: | 5 µl |
| Detection: | |
| ELSD: | T_{evap.}: 110 °C, T_{neb.}: 90 °C, V_{N₂}: 1.2 L/min |
| (DAD | 205 nm (BW4, Ref, 550 nm BW 100) |
| | 230 nm (BW4, Ref, 550 nm BW 100) |

The peaks were detected by Evaporative Light Scattering Detector. β-cyclodextrin elutes at 12.3 min.

### 2.2 Results

### Effect of hydroxy-acids and salts

The effect of different hydroxy acids, hydroxy acid salts and sodium hydroxide on the suspension homogeneity and also on the chemical stability of cetirizine in the suspensions was studied.

The cetirizine and β-cyclodextrin suspensions were prepared by stirring aqueous cetirizine solution with cyclodextrin and with the additives. The results of suspension homogeneity were evaluated visually and the chemical stability of cetirizine in the suspensions are summarized in Table 2.2.

**Table 2.2 Summary of the effect of different additives on homogeneity of the cetirizine and β-cyclodextrin suspension**

| **Additive** | **Molar ratio CTZ : Additive** | **BCD content in the filtrate [mg/ml]** | **Suspension homogeneity compared to control** |
|---|---|---|---|
| - | - | 115 | control |
| NaOH | not calculated | 115 | no significant change |
| NaOH | not calculated | 125 | no significant change |
| K-Na-tartarate | 1 : 5 | 120 | better |
| Tartaric acid | 1 : 2 | 130 | better |
| Citric acid | 1 : 1 | 154 | no significant change |
| Na-citrate | 1 : 1 | not measured | better |
| Na-citrate | 1 : 2 | not measured | better |
| Na-citrate | 1 : 3 | not measured | better |
| Na-citrate | 1 : 4 | 135 | better |

### Stabilization of cetirizine and β-cyclodextrin suspension with polymeric additives

The suspension stabilization effect of different types of polymeric additives was studied. The weighed solid cetirizine, cyclodextrin and Na-citrate were dissolved in the solutions of polymeric additives of different concentration. After stirring the suspensions for 2 hours the samples were stored without stirring for 1-2 hours, subsequent the suspension homogeneity was visually studied. Table 2.3 shows the results.

Among the tested polymeric additives HPMC and CMC were effective in the stabilization of the suspension. The amount of the additive should be between 0.5-1% to achieve suspension stabilizing effect.

### 2.3. Conclusions

The observed chemical degradations affecting both β-cyclodextrin and cetirizine component of the binary formulation can be effectively suppressed by employing a hydroxy-acid salt (Na-citrate). Moreover, Na-citrate improves the suspension homogeneity of the reaction mixture prepared for spray-drying technology. The effective amount of this additive was determined (3-6% by weight), which seems to be technically feasible.

Besides hydroxy acids and salts colloidal stabilizer polymers were also investigated. The tested polymeric additives effectively enhance the suspension homogeneity. The best result was obtained by applying 1% carboxymethyl cellulose to the cetirizine, β-cyclodextrin and Na-citrate suspension. Hydroxypropyl-methyl cellulose was also very effective additive to stabilize the suspension.

### EXAMPLE 3

### Preparation of chewing gum comprising an inclusion complex comprising cyclodextrin and cetirizine

Two different chewing gums are prepared in lab scale, one comprising free cetirizine and the other comprising an inclusion complex of cetirizine and β-cyclodextrin.

### 3.1 Material and Methods

Both chewing gums comprise gum base, bulk sweetener and flavours. The ingredients of the two different chewing gums are shown in table 3.1.

**Table 3.1 Type and amount of ingredients in chewing gums**

| **Chewing gum** | **1** | | **2** | |
|---|---|---|---|---|
| **Ingredient** | g/400 g | % | g/400 g | % |
| **Gum base** | | | | |
| Gum base | 160 | 40 | 160 | 40 |

| **Bulk Sweetener** | | | | |
|---|---|---|---|---|
| Maltitol | 225 | 49 | 197 | 49 |

| **Active compound** | | | | |
|---|---|---|---|---|
| Free Cetirizine | 2.20 | 0.55 | | |
| Cetirizine/β-cyclodextrin complex (1:5) | | | 31.00 | 7.74 |

| **Other** | | | | |
|---|---|---|---|---|
| Twin sweet | 1.20 | 0.30 | 1.20 | 0.30 |
| Acesulfame | 0.36 | 0.09 | 0.36 | 0.09 |

| **Flavour** | | | | |
|---|---|---|---|---|
| Grape | 8.8 | 2.20 | 8.8 | 2.20 |
| Menthol | 2.0 | 0.50 | 2.0 | 0.50 |
| **Total** | 400 | 100 | 400 | 100 |

The chewing gum components were mixed in a mixer with strong horizontally placed Z-blades, which processes the components into a homogeneous gum mass. The mixer is heated to a temperature of approx. 50-60°C. The mixing process starts with mixing and melting the gum base for 3 minutes.

The bulk sweetener maltitol was then added and mixed for 4 minutes. Subsequent, the sweeteners TwinSweet^{®}, acesulfame K, the active ingredient (free Cetirizine dihydrochloride or inclusion complex of Cetirizine dihydrochloride and β-cyclodextrin) were added and the mixture was mixed for further 6 minutes. The grape flavour was then added and mixed for further 5 minutes. Total mixing time to produce a homogeneous gum mass with the chewing gum components was 20 minutes.

When the mixing was completed, the gum mass was taken out onto trays and rolled out to a 1-2 cm thick sheet. The chewing gum mass was subsequent cooled for approx. 15-20 minutes until the right texture of the gum mass is achieved.

The chewing gum core was formed into approx. 1 g/piece rectangular tablets by the rolling and scoring line. In order to prevent the chewing gum cores from sticking to rollers, the chewing gum mass was powdered with talc. The formed chewing gum cores are then cooled to room temperature and hardened on trays at controlled temperature and moisture conditions.

The formed and cooled chewing gum cores were then coated and polished with a coating suspension in a round stainless steel coating kettle that rotate during the coating and polishing process.

The coating suspension had the following composition:

| Xylitol | **58.2 %** |
|---|---|
| Mannitol | **10.6 %** |
| Gelatin | **1.2 %** |
| Titanium dioxide | **0.9 %** |
| Acesulfame K | **0.15 %** |
| **Water** | **29.0 %** |

The coating suspension was produced by mixing and heated up to 70°C.

Cores of chewing gum were first separated manually before adding them into the coating kettle. Xylitol suspension was added in portions to the kettle and dispersed even over the surface of the cores after some time to smooth out. The operation is repeated until the tablet weight was reached about 1.3 g

Flavour (0.25 % of the total weight of the uncoated cores) was added during coating of the chewing gum product together with a small dosage of the coating suspension, when the tablet weight is between 1.2 g and 1.3 g.

The polishing also takes place in rotating kettles in which carnauba wax (0.1 % of total weight of the uncoated cores) is added to the coated cores in one portion. Polishing is done until a shinning surface is achieved, typically for 10-20 minutes.

After the preparation, the two chewing gums were stored up to 3 months at a temperature of 40°C and 75 RH (relative humidity). The amount of cetirizine was measured at the beginning of the storage (initial amount) and after 2 and 3 months.

### 3.2 Results

The result of the stability test (40°C/75%RH) showed that the formulation, after 2 and 3 months respectively, comprising an inclusion complex of cetirizine and β-cyclodextrin had a better stability than formulations comprising free cetirizine. The results are shown in the below table 3.2 and graphically shown in Figure 1.

**Table 3.2 Percentage of initial amount of cetirizine after storage of two different chewing gums**

| **Formulation** | **Initial amount (%)** | **2 months 40/75 (%)** | **3 months 40/75 (%)** |
|---|---|---|---|
| Free CTZ | 100 | 94.9 | **93.0** |
| **CTZ/β-CD** | **100** | **99.6** | **96.8** |

The results in table 2.3 show that the stability of cetirizine in the chewing gum during storage is improved with about 4-5% when complex-bound with β-cyclodextrin compared to cetirizine not complex-bound to β-cyclodextrin.

### EXAMPLE 4

### Preparation of compressed chewing gum comprising an inclusion complex comprising cyclodextrin and cetirizine

Different types of compressed chewing gum tablets comprising an inclusion complex comprising cyclodextrin and cetirizine are prepared. The chewing gum tablets are manufactured from a commercially available gum base (Comfree, available from Gumlink A/S, Vejle, Denmark) supplemented with about 15% by weight elastomer, about 20% by weight natural resin, about 20% by weight PVA, about 20% by weight filler, about 5% emulsifier, and about 20% by weight fat. Such mixture is in the following referred to as the "gum base"

### Inclusion complex between the chewing gum particles containing gum base

The gum base is feed to an extruder (Leistrits ZSE/BL 360 kw 104, available from GALA GmbH, Germany) and flavour is added and mixed to the gum base in the extruder. The resulting gum base composition is extruded to a granulator comprising a die plate and liquid filled chamber (A5 PAC 6, available from GALA GmbH, Germany) connected to a water system comprising water supply for the granulator and centrifugal dryer (TWS 20, available from GALA GmbH, Germany). The granulator produces chewing gum particles containing gum base. The preparation of chewing gum particles is described in details in EP1474993, EP147994 and EP147995.

The chewing gum particles containing gum base are subsequently mixed with the inclusion complex and/or other chewing gum ingredients.

### Inclusion complex in the chewing gum particles containing gum base

Method I: The gum base is feed to an extruder (Leistrits ZSE/BL 360 kw 104, available from GALA GmbH, Germany) and the inclusion complex and/or flavour are added and mixed to the gum base in the extruder. The resulting gum base composition is extruded to a granulator comprising a die plate and liquid filled chamber (A5 PAC 6, available from GALA GmbH, Germany).
Method II: The inclusion complex may also be incorporated into the particles by adding it during the manufacturing of the gum base. The inclusion complex is added during the mixing of the gum base ingredients preferably in the end of mixing. The gum base can subsequent be particulated by extrusion, pelletizing, milling, grinding or any other methods

### Compression

Before pressing, the mixture is passed through a standard horizontal vibration sieve removing particles larger than 2.6 mm. The mixture is subsequently passed to a standard tablet pressing machine comprising dosing apparatus (e.g. P 3200 C, available from Fette GmbH, Germany) and pressed into compressed chewing gum tablets having one compressed module. The filling depth is approximately 7.5 mm and the diameter 7.0 mm. The tablets are pre-compressed to 5.0 mm and then main compressed to 3.2 mm using a pressing pressure of 33.0 - 33.6 kN. There are 61 punches on the rotor, and the rotor speed used is 11 rpm. The individual compressed tablets have a weight of approx. 1.5 g.

### Chewing gum tablets with two modules

Chewing gums having two compressed modules may be prepared as follows: 2 g of a portion comprising an inclusion complex located in the chewing gum particles containing gum base or a potion comprising an inclusion complex located between the chewing gum particles containing gum base is filed into the tablet pressing machine and compressed onto the above first module to form a chewing gum having two compressed modules. However, in some chewing gums, tablet material containing cetirizine may be used instead of chewing gum particles comprising gum base. Examples of such tablet materials are described above.

### EXAMPLE 5

### Compressed chewing gum tablet having two compressed modules

A compressed chewing gum tablet having two compressed modules comprising a first compressed module comprising chewing gum particles containing gum base and a second compressed module comprising an inclusion complex comprising cyclodextrin and cetirizine was prepared.

The portion for the first compressed module contained:

| | |
|---|---|
| Gum base, particles (with antioxidant BHT=700 ppm) | 400 g |
| Isomalt (for direct compression) | 513 g |
| Encapsulated Aspartame | 16 g |
| Acesulfam K | 1 g |
| Grapefruit flavour | 60 g |
| Magnesium stearate | 10 g |

The portion for the second compressed module contained:

| | |
|---|---|
| Cetirizine-β-cyclodextrin inclusion complex (Content of Cetirizine = 5 gram) | 80 g |
| Magnesium stearate | 0.8 g |
| Grapefruit flavour | 8.0 g |
| Sorbitol | 310.8 g |
| Saccharin sodium | 0.4 g |

The ingredients for each module were mixed dry in a conventional dry mixer and formed into a tablet in a two station tablet machine as described in Example 4. The mixtures gave a total of 1000 tablets where each tablet is made op of module 1 = 1000 mg and module 2 = 400 mg. The content of Cetirizine is 5 mg per chewing gum piece. If a chewing gum with 10 mg cetirizine is desired, 10 gram of cetirizine is added in the portion for the second module and the sorbitol content is adjusted to 230.8 gram.

### EXAMPLE 6

### Stability test of compressed chewing gum tablet having two compressed modules

Two compressed chewing gum tablets having two compressed modules comprising a first compressed module comprising chewing gum particles containing gum base and a second compressed module comprising cetirizine were prepared.

In chewing gum A the second module comprised an inclusion complex comprising cyclodextrin and cetirizine, and in chewing gum B the second module comprised free cetirizine.

### Chewing gum A+B

The portion for the first compressed module contained

| | |
|---|---|
| Gum base, particles (with antioxidant BHT=350 ppm) | 400 g |
| Isomalt (for direct compression) | 513 g |
| Encapsulated Aspartame | 16 g |
| Acesulfam K | 1 g |
| Grapefruit flavour | 60 g |
| Magnesium stearate | 10 g |

### Chewing gum A

The portion for the second compressed module contained

| | |
|---|---|
| Cetirizine-β-cyclodextrin inclusion complex (Content of Cetirizine = 10,25 gram) | 167 g |
| Magnesium stearate | 0.8 g |
| Grapefruit flavour | 8.0 g |
| Sorbitol | 223.8 g |
| Saccharin sodium | 0.4 g |

### Chewing gum B

The portion for the second compressed module contained

| | |
|---|---|
| Free cetirizine hydrochloride | 10.25 g |
| | |
| Magnesium stearate | 0.8 g |
| Grapefruit flavour | 8.0 g |
| Sorbitol | 380.55 g |
| Saccharin sodium | 0.4 g |

The ingredients for each module were mixed dry in a conventional dry mixer and formed into a tablet in a two station tablet machine as described in Example 4. The mixtures gave a total of 1000 tablets where each tablet is made op of module 1 = 1000 mg and module 2 = 400 mg.

The stability of the compressed chewing gum tablets was evaluated by the content of cetirizine and formation of impurities after storage at a temperature of 60°C.

**Table 6.1 Results of the stability test (60°C)**

| **Chewing gum A** | **Added** | **Initial** | **19 day's** |
|---|---|---|---|
| % cetirizine | 100 | 97 | 95 |
| Impurities total unknown | | <0.1 | 0.43 |
| Impurities total known | | <0.1 | 0.41 |
| Impurities total | | <0.1 | 0.84 |

**Table 6.2 Results of the stability test (60°C)**

| **Chewing gum B** | **Added** | **Initial** | **19 day's** |
|---|---|---|---|
| % cetirizine | 100 | 94 | 94 |
| Impurities total unknown % | | <0.1 | 4.24 |
| Impurities total known | | <0.1 | 0.67 |
| Impurities total | | <0.1 | 4.91 |

The results in tables 6.1 and 6.2 show that in the chewing gum comprising free cetirizine considerable more impurities are formed compared to in a chewing gum comprising an inclusion complex comprising cyclodextrin and cetirizine. This shows that cetirizine comprised in a cyclodextrin inclusion complex has a better stability compared to free cetirizine.

### EXAMPLE 7

### Stability test of compressed chewing gum tablet having two compressed modules

A compressed chewing gum tablet having two compressed modules comprising a first compressed module comprising chewing gum particles containing gum base and a second compressed module comprising an inclusion complex comprising cyclodextrin and cetirizine was prepared in a laboratory scale. The stability of cetirizine after storage was evaluated.

The portion for the first compressed module contained:

| | |
|---|---|
| Gum base, particles | 100.0 g |
| Isomalt | 131.75 g |
| Twinsweet | 0.75 g |
| Grapefruit flavour | 15.0 g |
| Magnesium stearate | 2.5 g |

The portion for the second compressed module contained:

| | |
|---|---|
| Cetirizine/β-cyclodextrin complex | 22.4 g |
| Grapefruit flavour | 2.0 g |
| Sorbitol | 75.3 g |
| Sodium saccharine | 0.1 g |
| Magnesium stearate | 0.2 g |

The ingredients of the first and second portion were separately mixed dry in a small container with round bottom.

The powder mixtures were subsequently dosed separately into a small manual compression machine and compressed with respectably 1-2 KN (layer one) and 12-15 KN (layer two), as described in Example 4.

The stability of the compressed chewing gum tablets was evaluated at a temperature of 40°C and 75% RH.

**Table 7.1 Results of the stability test (40°C and 75% RH)**

| **Initial** | **0.5 month** | **1 month** | **2 months** | **3 months** |
|---|---|---|---|---|
| 100% | 97.9% | 98.3% | 104.8% | 105.0% |

The results in table 7.1 show that the chewing gum formulation has an excellent stability.

### EXAMPLE 8

### The stability effect of the cetirizine/beta-cyclodextrin complex - part I

This following experiment was conducted to investigate the stability effects observed in Example 6, and particularly, to assess whether the improved stability, which was observed in Example 6, was caused by changes in the relative amount of sorbitol or by the formation of cetirizine/beta-cyclodextrin inclusion complexes.

Four powder samples were prepared with contents as described in Table 8.1.

**Table 8.1**

| | Free cetirizine 2HCl | Cetirizine/beta-cyclo-dextrin inclusion complex | Sorbitol |
|---|---|---|---|
| Sample 1 | | 164 mg^{*} | 224 mg |
| Sample 2 | | 164 mg^{*} | 380 mg |
| Sample 3 | 10 mg | | 224 mg |
| Sample 4 | 10 mg | | 380 mg |

| | | | |
|---|---|---|---|
| ^{*)} 164 mg Cetirizine/beta-cyclo-dextrin inclusion complex contains 10 mg cetirizine and 124 mg beta-cyclodextrin. The remaining 30 mg consists of residual water and auxiliary additives used in the complexation process (sodium-citrate and carboxymethyl cellulose). | | | |

The four samples were stored in capped 50 mL Polyethylene Duma^{™} containers (Superfos Pharma Pack, Denmark) at 40 degrees C and 75% relative humidity for 34 days, and were subsequently analysed by HPLC and the results are presented in Table 8.2.

**Table 8.2 Percentage of impurities relative to the original cetirizine contents.**

| | Sample 1 | Sample 2 | Sample 3 | Sample 4 |
|---|---|---|---|---|
| **Known impurities** | <0.1 | <0.1 | <0.1 | <0.1 |

| **Unknown imp.:** | | | | |
|---|---|---|---|---|
| CTZ-sorbitol ester | 0.29 | 0.49 | 8.87 | 10.98 |
| Other unknown imp. | 0.30 | 0.49 | 0.53 | 0.58 |
| **Total unknown imp.** | 0.59 | 0.98 | 9.39 | 11.56 |
| **Total impurities** | 0.59 | 0.98 | 9.39 | 11.56 |

The results of the Table 8.2 clearly show that beta-cyclodextrin-complexation of cetirizine leads to a 95-97% reduction of cetirizine-sorbitol ester impurities, relative to uncomplexed cetirizine, independent of the amount of sorbitol. The same effect was observed in Example 6, where the amount of "Impurities total unknown", which primarily consists of the cetirizine-sorbitol esters, was significantly reduced by using beta-cyclodextrin-complexed cetirizine (see Table 6.2 compared to Table 6.1). The effect of reducing the amount of sorbitol is small compared to the complexation effect.

### EXAMPLE 9

### The stability effect of the cetirizine/beta-cyclodextrin complex- part II

The following experiments were performed to assess whether the complexation between cetirizine and beta-cyclodextrin is necessary to obtain an improved stability or whether it is sufficient to have uncomplexed cyclodextrin and cetirizine in the same formulation.

Four powder samples were prepared with contents as described in Table 9.1.

**Table 9.1**

| | Free cetirizine 2HCl | Cetirizine/beta-cyclo-dextrin inclusion complex | Sorbitol | Beta-cyclodextrin |
|---|---|---|---|---|
| Sample 5 | | 164 mg^{*} | 224 mg | |
| Sample 6 | | 164 mg^{*} | 380 mg | |
| Sample 7 | 10 mg | | 224 mg | 124 mg |
| Sample 8 | 10 mg | | 380 mg | 124 mg |

| | | | | |
|---|---|---|---|---|
| ^{*)} 164 mg Cetirizine/beta-cyclo-dextrin inclusion complex contains 10 mg cetirizine and 124 mg beta-cyclodextrin. The remaining 30 mg consists of residual water and auxiliary additives used in the complexation process (sodium-citrate and carboxymethyl cellulose). | | | | |

The four samples were stored in capped 50 mL Polyethylene Duma^{™} containers (Superfos Pharma Pack, Denmark) at 40 degrees C and 75% relative humidity for 34 days, and were subsequently analysed by HPLC. The results are presented in Table 9.2.

**Table 9.2 Percentage of impurities relative to the original cetirizine contents.**

| | Sample 5 | Sample 6 | Sample 7 | Sample 8 |
|---|---|---|---|---|
| **Known impurities** | <0.1 | <0.1 | <0.1 | 0.4 |

| **Unknown imp.:** | | | | |
|---|---|---|---|---|
| CTZ-sorbitol ester | 0.29 | 0.49 | 10.44 | 9.93 |
| Other unknown imp. | 0.30 | 0.49 | 0.72 | 0.48 |
| **Total unknown imp.** | 0.59 | 0.98 | 11.15 | 10.41 |
| **Total impurities** | 0.59 | 0.98 | 11.15 | 10.78 |

The results in Table 9.2 show that un-complexed cyclodextrin does not have any significant effect on the stability of cetirizine. Formation of the inclusion complex is necessary for obtaining the improved stability.

### REFERENCES

Coleman, A. et al. 1992; J. Incl. Phen. Mol. Recogn in Chemistry 13. 2. 139-143. 1992
Duan Matt. et al Int.J. Pharm. 297.213-222.2005
Loftsson T. et al, J. Pharm. Sci. 93.5. 1091-1099. 2004
Masson, M et al Chem. Pharm. Bull. 53.8. 958-964. 2005

## Claims

1. A chewing gum comprising a gum base and at least one inclusion complex comprising a cyclodextrin and an active compound selected from 2-[4-(diphenylmethyl)-1-piperazine] derivatives having the general formula I: 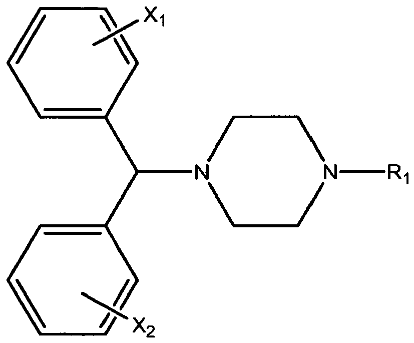 wherein
R₁ is selected from the group consisting of -CH₂CH₂-O-CH₂-R₂, -CH₂CH=CH-Ar₁, -CH₂-Ar₂ and 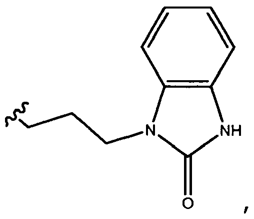 R₂ may be selected from the group consisting of a -CH₂OH group, a -COOH group and a -CONH₂ group;
Ar₁ and Ar₂ are independently an aromatic or heteroaromatic ring with 5 or 6 atoms in the ring, said heteroaromatic ring having 1, 2 or 3 heteroatoms selected from the group consisting of nitrogen, oxygen and sulfur, said ring being unsubstituted or substituted with C₁₋₄ alkyl, preferably methyl or tertiary butyl, Ar₁ and Ar₂ preferably being unsubstituted phenyl or phenyl substituted with C₁₋₄ alkyl, preferably methyl or tertiary butyl, and
X₁ and X₂ may independently be selected from the group consisting of a hydrogen atom, a halogen atom, a straight-chain or branched C₁-C₄ alkoxy group or a trifluoromethyl group; as well as pharmaceutically acceptable salts, geometrical isomers, enantiomers, diastereomers and mixtures thereof.

2. The chewing gum according to claim 1, wherein the active compound according to formula I is selected from the group consisting of buclizine, cetirizine, chlorcyclizine, cinnarizine, cyclizine, hydroxyzine, levocetirizine, meclozine, efletirizine and oxatomide, as well as any pharmaceutically acceptable salts, geometrical isomers, enantiomers, diastereomers and mixtures thereof.

3. The chewing gum according to any one of the preceding claims, wherein the active compound according to formula I is cetirizine or a salt thereof, preferably cetirizine dihydrochloride.

4. The chewing gum according to any one of the preceding claims, wherein at least one cyclodextrin is selected from the group consisting of alpha-cyclodextrin, beta-cyclodextrin, gamma-cyclodextrin, and derivatives thereof.

5. The chewing gum according to claim 4, wherein at least one cyclodextrin is selected from the group consisting of heptakis (2,6-di-o-methyl)-beta-cyclodextrin, randomly methylated beta-cyclodextrin and hydroxypropyl beta-cyclodextrin.

6. The chewing gum according to any one of the preceding claims, wherein at least one the cyclodextrin is beta-cyclodextrin.

7. The chewing gum according to any of the preceding claims, wherein the chewing gum comprises a first compressed module comprising compressed chewing gum particles containing gum base.

8. The chewing gum according to any of the preceding claims, wherein the stoichiometric ratio between the compound(s) according to formula I and cyclodextrin of said inclusion complex is at most 1:1.

9. The chewing gum according to any one of the preceding claims, wherein the inclusion complex is present in an amount of at least 0.2% by weight of the uncoated chewing gum.

10. The chewing gum according to any of the preceding claims, wherein the molar ratio between the total amount of compound according to formula I and the total amount of cyclodextrin is at the most 1:1.

11. The chewing gum according to any one of the preceding claims, wherein the total amount of cyclodextrin is present in an amount of at least 0.03% by weight of the uncoated chewing gum.

12. The chewing gum according to any one of the preceding claims, wherein the amount of complex-bound active compound according to formula I is at least 20% by weight of the total amount of compound according to formula I present in said chewing gum.

13. The chewing gum according to any one of the preceding claims, furthermore comprising a bulk sweetener.

14. A method of preparing a chewing gum comprising at least one inclusion complex comprising a cyclodextrin and one or more active compound(s) according to formula I, the method comprising the steps of:
a) providing at least one inclusion complex comprising a cyclodextrin and a compound according to formula I;
b) providing a gum base;
c) optionally providing one or more further chewing gum ingredients,
d) mixing said inclusion complex and gum base, and optionally the one and more further chewing gum ingredients, and thus obtaining a mixture; and
e) shaping the mixture to obtain the chewing gum.

15. The method of preparing a compressed chewing gum comprising at least one inclusion complex comprising a cyclodextrin and one or more active compound(s) according to formula I having one compressed module, the method comprising the steps of:
a) providing a portion comprising at least one inclusion complex comprising a cyclodextrin and one or more active compound(s) according to formula I, and chewing gum particles containing gum base,
b) optionally providing one or more further chewing gum ingredients,
c) dosing the portion comprising the at least one inclusion complex comprising a cyclodextrin and one or more active compound(s) according to formula I, and chewing gum particles containing gum base, and optionally the one or more further chewing gum ingredients, and
d) compressing a) and b) after dosing to obtain a first compressed module.

16. A method of preparing a chewing gum comprising at least one inclusion complex comprising a cyclodextrin and one or more active compound(s) according to formula I having two compressed modules, the method comprising the steps of:
a) providing chewing gum particles containing gum base and optionally portion(s) comprising one or more chewing gum ingredients,
b) providing a portion comprising tablet material and a portion comprising at least one inclusion complex comprising a cyclodextrin and one or more active compound(s) according to formula I,
c) compressing a), to obtain a first compressed module,
d) contacting the first compressed module with b),
e) compressing b) and the first compressed module, to obtain a coherent compressed chewing gum comprising a first compressed module and a second compressed module.

17. Use of cyclodextrin to protect one or more active compound(s) according to formula I against degradation in a chewing gum, wherein said cyclodextrin forms an inclusion complex with said compound(s).

## Patentansprüche

1. Kaugummi, enthaltend eine Gummibase und mindestens einen Einschlusskomplex, der ein Cyclodextrin und eine aktive Verbindung enthält, die ausgewählt ist aus 2-[4-(Diphenylmethyl)-1-piperazin]-Derivaten mit der allgemeinen Formel I: 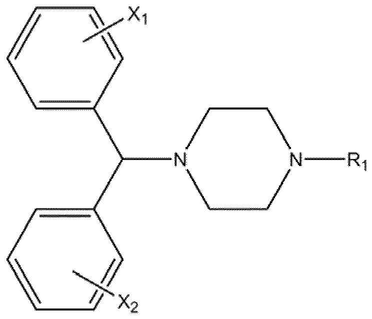 wobei
R₁ ausgewählt ist aus der Gruppe bestehend aus -CH₂CH₂-O-CH₂-R₂, -CH₂CH=CH-Ar₁, -CH₂-Ar₂ und 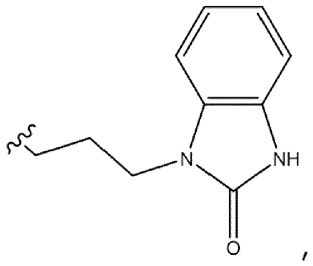 R₂ ausgewählt sein kann aus der Gruppe bestehend aus einer -CH₂OH-Gruppe, einer -COOH-Gruppe und einer -CONH₂-Gruppe;
Ar₁ und Ar₂ unabhängig ein aromatischer oder heteroaromatischer Ring mit 5 oder 6 Atomen im Ring sind, welcher heteroaromatische Ring 1, 2 oder 3 Heteroatome hat, die ausgewählt sind aus der Gruppe bestehend aus Stickstoff, Sauerstoff und Schwefel, welcher Ring unsubstituiert oder mit C₁₋₄-Alkyl, vorzugsweise Methyl oder tertiärem Butyl substituiert ist, wobei Ar₁ und Ar₂ vorzugsweise unsubstituiertes Phenyl oder mit C₁₋₄-Alkyl, vorzugsweise Methyl oder tertiärem Butyl substituiertes Phenyl sind, und
X₁ und X₂ unabhängig ausgewählt sein können aus der Gruppe bestehend aus einem Wasserstoffatom, einem Halogenatom, einer geradkettigen oder verzweigten C₁-C₄-Alkoxygruppe oder einer Trifluormethylgruppe; sowie pharmazeutisch akzeptable Salze, geometrische Isomere, Enantiomere, Diastereomere und Mischungen daraus.

2. Kaugummi nach Anspruch 1, bei welchem die aktive Verbindung gemäß Formel I ausgewählt ist aus der Gruppe bestehend aus Buclizin, Cetirizin, Chlorcyclizin, Cinnarizin, Cyclizin, Hydroxyzin, Levocetirizin, Meclozin, Efletirizin und Oxatomid sowie beliebigen pharmazeutisch akzeptablen Salzen, geometrischen Isomeren, Enantiomeren, Diastereomeren und Mischungen daraus.

3. Kaugummi nach einem der vorhergehenden Ansprüche, bei welchem die Verbindung gemäß Formel I Cetirizin oder ein Salz davon, vorzugsweise Cetirizindihydrochlorid ist.

4. Kaugummi nach einem der vorhergehenden Ansprüche, bei welchem mindestens ein Cyclodextrin ausgewählt ist aus der Gruppe bestehend aus alpha-Cyclodextrin, beta-Cyclodextrin, gamma-Cyclodextrin und Derivaten davon.

5. Kaugummi nach Anspruch 4, bei welchem mindestens ein Cyclodextrin ausgewählt ist aus der Gruppe bestehend aus Heptakis-(2,6-di-o-methyl)-betacyclodextrin, zufällig methyliertem beta-Cyclodextrin und Hydroxypropyl-beta-Cyclodextrin.

6. Kaugummi nach einem der vorhergehenden Ansprüche, bei welchem das mindestens eine Cyclodextrin beta-Cyclodextrin ist.

7. Kaugummi nach einem der vorhergehenden Ansprüche, bei welchem der Kaugummi ein erstes komprimiertes Modul aufweist, das komprimierte Kaugummipartikel aufweist, die Gummibase enthalten.

8. Kaugummi nach einem der vorhergehenden Ansprüche, bei welchem das stöchiometrische Verhältnis zwischen der bzw. den Verbindung(en) gemäß Formel I und Cyclo-dextrin des Einschlusskomplexes höchstens 1:1 ist.

9. Kaugummi nach einem der vorhergehenden Ansprüche, bei welchem der Einschlusskomplex in einer Menge von mindestens 0,2 Gew.-% des unbeschichteten Kaugummis vorhanden ist.

10. Kaugummi nach einem der vorhergehenden Ansprüche, bei welchem das molare Verhältnis zwischen der Gesamtmenge der Verbindung gemäß Formel I und der Gesamtmenge von Cyclodextrin höchstens 1:1 ist.

11. Kaugummi nach einem der vorhergehenden Ansprüche, bei welchem die Gesamtmenge von Cyclodextrin in einer Menge von mindestens 0,03 Gew.-% des unbeschichteten Kaugummis vorhanden ist.

12. Kaugummi nach einem der vorhergehenden Ansprüche, bei welchem die Menge der Komplex-gebundenen aktiven Verbindung gemäß Formel I mindestens 20 Gew.-% der Gesamtmenge der Verbindung gemäß Formel I ist, die in dem Kaugummi vorhanden ist.

13. Kaugummi nach einem der vorhergehenden Ansprüche, ferner enthaltend einen Füllsüßstoff.

14. Verfahren zur Herstellung eines Kaugummis, der mindestens einen Einschlusskomplex enthält, der ein Cyclodextrin und eine oder mehrere aktive Verbindung(en) gemäß der Formel I enthält, welches Verfahren die Schritte enthält:
a) Bereitstellen mindestens eines Einschlusskomplexes, der ein Cyclodextrin und eine Verbindung gemäß der Formel I enthält;
b) Bereitstellen einer Gummibase;
c) optional Bereitstellen eines oder mehrerer weiterer Kaugummiinhaltsstoffe;
d) Mischen des Einschlusskomplexes und der Gummibase und optional des einen oder der mehreren weiteren Kaugummiinhaltsstoffe, so dass eine Mischung erhalten wird; und
e) Formen der Mischung, um den Kaugummi zu erhalten.

15. Verfahren zur Herstellung eines komprimierten Kaugummis, der mindestens einen Einschlusskomplex enthält, der ein Cyclodextrin und eine oder mehrere aktive Verbindung(en) gemäß der Formel I enthält, der ein komprimiertes Modul hat, welches Verfahren die Schritte enthält:
a) Bereitstellen eines Anteils, der mindestens einen Einschlusskomplex aufweist, der ein Cyclodextrin und eine oder mehrere aktive Verbindung(en) gemäß der Formel I enthält sowie Kaugummipartikel, die Gummibase enthalten,
b) optional Bereitstellen eines oder mehrerer weiterer Kaugummiinhaltsstoffe,
c) Dosieren des Anteils, der den mindestens einen Einschlusskomplex aufweist, der ein Cyclodextrin und eine oder mehrere aktive Verbindung(en) gemäß der Formel I enthält sowie Kaugummipartikel, die Gummibase enthalten, und optional den einen oder die mehreren weiteren Kaugummiinhaltsstoffe, und
d) Komprimieren von a) und b) nach der Dosierung, um ein erstes komprimiertes Modul zu erhalten.

16. Verfahren zur Herstellung eines Kaugummis, der mindestens einen Einschlusskomplex enthält, der ein Cyclodextrin und eine oder mehrere aktive Verbindung(en) gemäß der Formel I enthält, der zwei komprimierte Module hat, welches Verfahren die Schritte enthält:
a) Bereitstellen von Kaugummipartikeln, die Gummibase enthalten, und optional von einem oder mehreren Anteilen, die einen oder mehrere Kaugummiinhaltsstoffe enthalten,
b) Bereitstellen eines Anteils, der Tablettenmaterial enthält, und eines Anteils, der mindestens einen Einschlusskomplex aufweist, der ein Cyclodextrin und eine oder mehrere aktive Verbindung(en) gemäß der Formel I enthält,
c) Komprimieren von a), um ein erstes komprimiertes Modul zu erhalten,
d) Kontaktieren des ersten komprimierten Moduls mit b),
e) Komprimieren von b) und des ersten komprimierten Moduls, um einen kohärenten komprimierten Kaugummi zu erhalten, der ein erstes komprimiertes Modul und ein zweites komprimiertes Modul enthält.

17. Verwendung von Cyclodextrin zum Schutz einer oder mehrerer aktiver Verbindung(en) gemäß der Formel I gegen die Zersetzung in einem Kaugummi, wobei das Cyclodextrin einen Einschlusskomplex mit der bzw. den Verbindung(en) bildet.

## Revendications

1. Gomme à mâcher comprenant une gomme base et au moins un complexe d'inclusion comprenant une cyclodextrine et un composé actif choisi parmi les dérivés de 2-[4-(diphénylméthyl)-1-pipérazine] de formule générale I : 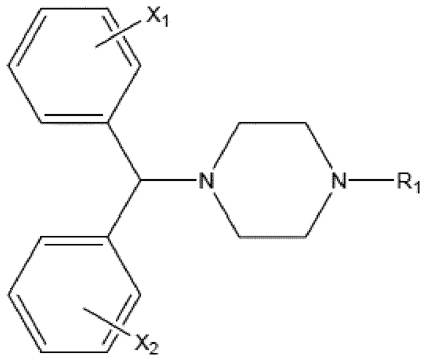 dans lequel
R₁ est choisi dans le groupe constitué par -CH₂CH₂-O-CH₂-R₂, -CH₂CH=CH-Ar₁, -CH₂Ar₂ et 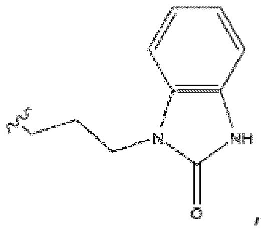 R₂ peut être choisi dans le groupe constitué par un groupe -CH₂OH, un groupe -COOH et un groupe -CONH₂ ;
Ar₁ et Ar₂ sont indépendamment l'un de l'autre un cycle aromatique ou hétéroaromatique ayant 5 ou 6 atomes dans le cycle, ledit cycle hétéroaromatique ayant 1, 2 ou 3 hétéroatomes choisis dans le groupe constitué par l'azote, l'oxygène et le soufre, ledit cycle étant non substitué ou substitué par de l'alkyle en C₁-₄, de préférence du méthyle ou du butyle tertiaire, Ar₁ et Ar₂ étant de préférence du phényle non substitué ou du phényle substitué par de l'alkyle en C₁-₄, de préférence du méthyle ou du butyle tertiaire, et
X₁ et X₂ peuvent être indépendamment l'un de l'autre choisis dans le groupe constitué par un atome d'hydrogène, un atome d'halogène, un groupe alcoxy en C₁-₄ linéaire ou ramifié ou un groupe trifluorométhyle ; ainsi que les sels, isomères géométriques, énantiomères, diastéréomères pharmaceutiquement acceptables et mélanges de ceux-ci.

2. Gomme à mâcher selon la revendication 1, dans laquelle le composé actif selon la formule I est choisi dans le groupe constitué par la buclizine, la cétirizine, la chlorcyclizine, la cinnarizine, la cyclizine, l'hydroxyzine, la lévocétirizine, la méclozine, l'eflétirizine et l'oxatomide, ainsi que tout sel, isomère géométrique, énantiomère, diastéréomère pharmaceutiquement acceptable et mélange de ceux-ci.

3. Gomme à mâcher selon l'une quelconque des revendications précédentes, dans laquelle le composé actif selon la formule I est la cétirizine ou un sel de celle-ci, de préférence du dichlorhydrate de cétirizine.

4. Gomme à mâcher selon l'une quelconque des revendications précédentes, dans laquelle au moins une cyclodextrine est choisie dans le groupe constitué par l'alpha-cyclodextrine, la bêta-cyclodextrine, la gamma-cyclodextrine et les dérivés de celles-ci.

5. Gomme à mâcher selon la revendication 4, dans laquelle au moins une cyclodextrine est choisie dans le groupe constitué par l'heptakis-(2,6-di-O-méthyl)-bêta-cyclodextrine, la bêta-cyclodextrine méthylée de façon aléatoire et l'hydroxypropyl-bêta-cyclodextrine.

6. Gomme à mâcher selon l'une quelconque des revendications précédentes, dans laquelle au moins une cyclodextrine est de la bêta-cyclodextrine.

7. Gomme à mâcher selon l'une quelconque des revendications précédentes, dans laquelle la gomme à mâcher comprend un premier module comprimé comprenant des particules de gomme à mâcher comprimées contenant de la gomme base.

8. Gomme à mâcher selon l'une quelconque des revendications précédentes, dans laquelle le rapport stoechiométrique entre le(s) composé(s) selon la formule I est la cyclodextrine dudit complexe d'inclusion est au maximum de 1:1.

9. Gomme à mâcher selon l'une quelconque des revendications précédentes, dans laquelle le complexe d'inclusion est présent dans une quantité d'au moins 0,2 % en masse de la gomme à mâcher non enrobée.

10. Gomme à mâcher selon l'une quelconque des revendications précédentes, dans laquelle le rapport molaire entre la quantité totale du composé selon la formule I et la quantité totale de cyclodextrine est au maximum de 1:1.

11. Gomme à mâcher selon l'une quelconque des revendications précédentes, dans laquelle la cyclodextrine est présente dans une quantité totale d'au moins 0,03 % en masse de la gomme à mâcher non enrobée.

12. Gomme à mâcher selon l'une quelconque des revendications précédentes, dans laquelle la quantité du composé actif lié au complexe selon la formule I est au moins de 20 % en masse de la quantité totale du composé selon la formule I présent dans ladite gomme à mâcher.

13. Gomme à mâcher selon l'une quelconque des revendications précédentes, comprenant en outre un édulcorant de charge.

14. Procédé de préparation d'une gomme à mâcher comprenant au moins un complexe d'inclusion comprenant une cyclodextrine et un ou plusieurs composé(s) actif(s) selon la formule I, le procédé comprenant les étapes consistant à :
a) fournir au moins un complexe d'inclusion comprenant une cyclodextrine et un composé selon la formule I ;
b) fournir une gomme base ;
c) fournir facultativement un ou plusieurs autre(s) ingrédient(s) pour gomme à mâcher ;
d) mélanger ledit complexe d'inclusion et ladite gomme base, et facultativement le ou les différents autre(s) ingrédient(s) pour gomme à mâcher, et obtenir ainsi un mélange ; et
e) former le mélange de façon à obtenir la gomme à mâcher.

15. Procédé de préparation d'une gomme à mâcher comprimée comprenant au moins un complexe d'inclusion comprenant une cyclodextrine et un ou plusieurs composé(s) actif(s) selon la formule I ayant un module comprimé, le procédé comprenant les étapes consistant à :
a) fournir une partie comprenant au moins un complexe d'inclusion comprenant une cyclodextrine et un ou plusieurs composé(s) actif(s) selon la formule I, et des particules de gomme à mâcher contenant de la gomme base,
b) fournir facultativement un ou plusieurs autre(s) ingrédient(s) pour gomme à mâcher ;
c) doser la partie comprenant l'au moins un complexe d'inclusion comprenant une cyclodextrine et un ou plusieurs composé(s) actif(s) selon la formule I, et des particules de gomme à mâcher contenant de la gomme base, et facultativement le ou les différents autre(s) ingrédient(s) pour gomme à mâcher, et
d) comprimer a) et b) après dosage pour obtenir un premier module comprimé.

16. Procédé de préparation d'une gomme à mâcher comprenant au moins un complexe d'inclusion comprenant une cyclodextrine et un ou plusieurs composé(s) actif(s) selon la formule I ayant deux modules comprimés, le procédé comprenant les étapes consistant à :
a) fournir des particules de gomme à mâcher contenant de la gomme base, et facultativement une ou des partie(s) comprenant un ou plusieurs ingrédient(s) pour gomme à mâcher,
b) fournir une partie comprenant un matériau de type comprimé et une partie comprenant au moins un complexe d'inclusion comprenant une cyclodextrine et un ou plusieurs composé(s) actif(s) selon la formule I,
c) comprimer a) pour obtenir un premier module comprimé,
d) mettre en contact le premier module comprimé avec b),
e) comprimer b) et le premier module comprimé pour obtenir une gomme à mâcher comprimée cohérente comprenant un premier module comprimé et un deuxième module comprimé.

17. Utilisation de cyclodextrine pour protéger un ou plusieurs composé(s) actif(s) selon la formule I contre la dégradation dans une gomme à mâcher, dans laquelle ladite cyclodextrine forme un complexe d'inclusion avec le(s)dit(s) composé(s).
